# EUROPEAN PATENT APPLICATION

(11) **EP 4 265 741 A1**
(43) Date of publication of application: **25.10.2023**
(21) Application number: 22382374.1
(22) Date of filing: 21.04.2022
(51) Int. Cl.: C12Q 1/70, C12N 9/22, C12Q 1/6818, C12Q 1/6883

(54) **MULTIPLEXABLE CRISPR-CAS9-BASED VIRUS DETECTION METHOD**

(71) Applicant: Consejo Superior de Investigaciones Científicas (CSIC), 28006 Madrid (ES); Universitat de València, 46010 Valencia (ES)
(72) Inventor: Rodrigo Tárrega, Guillermo, Madrid (ES); Márquez Costa, Rosa, Madrid (ES); Montagud Martínez, Roser, Madrid (ES); Ruiz González, Raúl, Madrid (ES); Heras Hernández, María, Madrid (ES)
(74) Representative: Pons

(57) **Abstract**

The present invention belongs to the field of biomedicine and viral diagnosis. In particular, the invention relates to a method for *in vitro* virus detection and/or *in vitro* specific viral sequence detection, including without limitation to, SARS-CoV-2 detection, based on the use of a CRISPR-Cas9 system.

## Description

The present invention belongs to the field of biomedicine and viral diagnosis. In particular, the invention relates to a method for *in vitro* virus detection and/or *in vitro* specific viral sequence detection, including without limitation to, SARS-CoV-2 detection.

### BACKGROUND ART

Coronavirus disease 2019 (COVID-19) pandemic caused by severe acute respiratory syndrome coronavirus 2 (SARS-CoV-2) has highlighted the challenges in viral infection diagnosis. A fast and confident diagnosis contributes to significantly reduce virus transmission in the community and allows early therapeutic actions that can mitigate acute outcomes of infection. Currently, reverse transcription quantitative polymerase chain reaction (RT-qPCR) is the gold-standard diagnostic technique of infectious diseases in the clinic due to its high sensitivity and specificity. However, when a rapid and massive intervention is required, such as in a pandemic context, alternative techniques that can bypass, at least in part, the need for expensive equipment and well-trained personnel are required.

Clustered regularly interspaced short palindromic repeats (CRISPR) systems are being repurposed in recent years for diagnostic applications (Gootenberg JS, et al., Science 356, 438-442 (2017); Chen JS, et al., Science 360, 436-439 (2018)). Owing to the ability of some CRISPR-associated (Cas) proteins to display a collateral catalytic activity upon target recognition, sensitive and specific nucleic acid detection has been proven possible. In combination with isothermal amplification techniques (Zhao Y. et al., Chem Rev 115, 12491-12545 (2015)), a detection with a sensitivity at the attomolar scale (i.e., about one copy per microliter) and a sensitivity at one nucleotide resolution has been achieved, atop of bypassing the dependence on RT-qPCR equipment. In this regard, CRISPR systems may represent a suitable alternative for infectious diseases diagnosis.

Several CRISPR-Cas based virus detection methods, including multiplex detection, are disclosed in the state of the art.

WO2021222878A1 discloses methods for the detection of nucleotide sequences including SARS-CoV-2 detection based on CRISPR-Cas9. Particularly, Cas9 diagnosis for single and multiplexed targets, is mediated by the Cas9 nuclease properties, that holds potential to serve as a diagnostic platform by cleaving a fluorescent probe in a sequence-specific manner. COVID-19-specific probes labelled with a fluorescent marker and quencher when hybridizes with SARS-CoV-2 amplicons, are cleaved by Cas9 nuclease together with a COVID-19 sgRNA.

Methods for detecting the level or existence of pathogens predefined nucleic acid sequences, including SARS-CoV-2, based on CRISPR-associated nucleases are disclosed in WO2021216573A1. Particularly, some of the methods comprise contacting a nucleotide sequence to form a mixture with a CRISPR-associated nuclease, a gRNA, and a reporter molecule. The reporter molecule includes a detectable tag and a nucleotide sequence, wherein said nucleotide sequence is collaterally cleaved by the CRISPR-associated nuclease upon recognition of the target sequence by the gRNA and activation of the CRISPR-associated nuclease. Thus, the detection is mediated by the reporter molecule cleavage.

However, multiplex diagnosis remains challenging due to limitations such as the non-specificity of Cas9 collateral catalytic activity. To overcome this limitation, orthogonal Cas proteins can be employed to achieve multiplexed nucleic acid detection in a single reaction. Nevertheless, the number of nucleic acids that can be detected simultaneously is limited by the number of different Cas proteins involved in the assay.

Therefore, in the light of the state of the art, there is a need to find alternative virus detection methods, especially in the field of multiplex and simultaneous virus detection.

### DESCRIPTION OF THE INVENTION

The present invention discloses a method for *in vitro* virus detection and/or *in vitro* specific viral sequence detection based on a CRISPR-Cas9 system and the use of molecular beacons, allowing simple and multiplex virus detection and/or specific viral sequence/s detection in a sample.

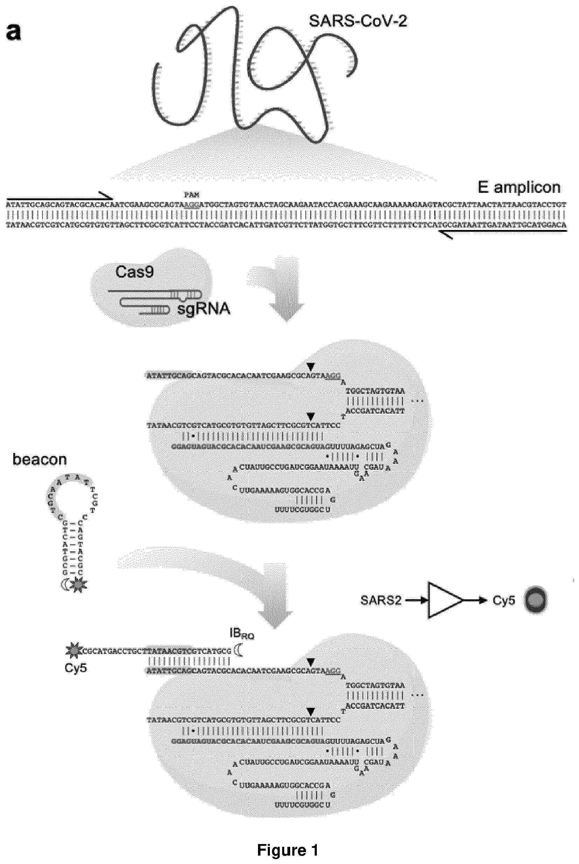

The inventors have developed this method based on the strand displacement Cas9 action, rather than on collateral catalysis, and the use of molecular beacons specifically designed to hybridize to a strand-displaced region of an amplicon previously amplified and recognised by appropriates sgRNAs (Figure 1a).

Inventors have applied this approach in the detection of SARS-CoV-2 (Figure 1), simultaneous detection of three different SARS-CoV-2 genomic regions (Figure 2b and 2c), and simultaneous detection of three different coronavirus in a sample (Figure 3b and 3c).

This way, the present invention provides an alternative method for *in vitro* virus detection, having several associated advantages such as: (i) the method allows multiplex virus detection in a single sample; (ii) it is based on strand displacement Cas9 action, rather than on the collateral one, overcoming limitations in the field of multiplex diagnostics due to the non-specificity of collateral activity.; and (iii) the sgRNA-Cas9 ribonucleoprotein gives the specificity of the detection, and the interaction between the displaced DNA strand and molecular beacon gives the fluorescence readout. Consequently, different DNA amplicons can be detected with the same nuclease (Cas9).

### Method of the invention

Thus, an aspect of the present invention relates to a method for *in vitro* virus detection and/or *in vitro* specific viral sequence detection, hereinafter the "method of the invention", comprising the following steps:
(a) amplifying, at least, one target viral nucleotide sequence from a sample, obtaining, at least, one amplicon with a protospacer adjacent motif (PAM),
(b) contacting the amplicon obtained in the step (a) to a Cas9 protein and, at least, one sgRNA, wherein the sgRNA comprises a nucleotide sequence to hybridize with target region of the amplicon obtained in (a), obtaining, at least, one ribonucleoprotein-DNA complex,
(c) adding, at least, one molecular beacon to the ribonucleoprotein-DNA complex obtained in (b), wherein the molecular beacon comprises a nucleotide sequence that hybridizes with a strand-displaced region of the amplicon, a fluorophore linked to the 3' or 5' end of its nucleotide sequence, and a quencher linked to the remaining end of its nucleotide sequence, arranged in a conformation, such that:
   (i) when the molecular beacon does not hybridize to the strand-displaced region of the amplicon, the molecular beacon is arranged in a hairpin structure, with the quencher and fluorophore located spatially close without emitting fluorescence or emitting a basal fluorescence;
   (ii) when the molecular beacon hybridizes to the strand-displaced region of the amplicon, the molecular beacon is arranged linearly, with the quencher and fluorophore being spatially separated, emitting fluorescence, and
(d) measuring, at least, one fluorescent signal and comparing to control values, wherein an increase of the fluorescent signal compared to control values indicates that the target viral nucleotide sequence is present in the sample, hence allowing virus detection and/or specific viral sequence detection.

The phrase "strand-displaced region of the amplicon", refers to a region of the amplicon strand which has been displaced by the Cas9 strand-displacement activity (Fig. 1a). Cas9 strand-displacement activity is an enzymatic activity that produces the displacement of the non-complementary strand of the target nucleotide sequence. In the present invention, upon the complex comprised by the Cas9 protein and a sgRNA is formed, the complex is directed to a specific DNA target region (in the present invention, an amplicon target) by a sgRNA specific nucleotide sequence. This sgRNA sequence hybridizes with the complementary strand of the DNA, displacing the noncomplementary strand and forming an R-loop.

In the present invention, the interaction of the beacon with the displaced strand causes a change of its arrangement (opening or linearization), thereby producing a fluorescent signal that can be detected. This interaction may be seeded by the pairing of some nucleotides located in the loop of the beacon with the complementary nucleotides located in 3' or 5' end of the displaced strand.

### Step (a): amplification step

A first step of the method of the invention [step (a)], comprises amplifying, at least, one target viral nucleotide sequence from a sample, obtaining, at least, one amplicon which comprises a protospacer adjacent motif (PAM).

In the present invention, the term "amplifying", refers to the process of obtaining, at least, one amplicon from, at least, one target viral nucleotide sequence. Amplification methods for obtaining the amplicon are well known in the state of the art. Examples of amplification methods include methods for generating a double-stranded DNA (dsDNA) molecule as amplified material, such as, but without limitation to, polymerase chain reactions (PCR), including qPCR, recombinase polymerase amplification (RPA), or loop-mediated isothermal amplification (LAMP).

Thus, in a preferred embodiment of the method of the invention, the target viral nucleotide sequence is amplified by PCR, RPA, or LAMP.

As a person skilled in the art knows, the amplification may be followed by a purification step, in order to remove molecular elements that may interfere in the rest of the method, such as primers or enzymes. Thus, another embodiment of the method of the invention comprises an additional step, after step (a), comprising a purification of the amplified material/amplicon.

Amplification methods mentioned above are based on the use of primers. The primers may include any suitable primers that amplify target viral nucleotide sequence/s from viral nucleic acid/s. As a person skilled in the art knows, a forward primer and a reverse primer is used for the amplification of a nucleotide sequence to obtain a double-stranded nucleotide sequence, in the present invention an amplicon.

In a preferred embodiment of the method of the invention, a forward primer, which comprises, or consists of, a nucleotide sequence with a sequence identity of, at least 70, 75, 80, 85, 90, 91, 92, 93, 94, 95, 96, 97, 98 or 99% to SEQ ID NO: 1, and a reverse primer which comprises, or consists of, a nucleotide sequence with a sequence identity of , at least 70, 75, 80, 85, 90, 91, 92, 93, 94, 95, 96, 97, 98 or 99% to SEQ ID NO: 2 are used in the step (a).
SEQ ID NO: 1
   GACCCCAAAATCAGCGAAAT
SEQ ID NO: 2
   TCTGGTTACTGCCAGTTGAATCTG

In a more preferred embodiment of the step (a) of the method of the invention, the forward primer comprises, or consists of, the SEQ ID NO: 1, and the reverse primer comprises, or consists of, the SEQ ID NO: 2.

In another preferred embodiment of the method of the invention a forward primer, which comprises, or consists of, a nucleotide sequence with a sequence identity of, at least 70, 75, 80, 85, 90, 91, 92, 93, 94, 95, 96, 97, 98 or 99% to SEQ ID NO: 3, and a reverse primer which comprises, or consists of, a nucleotide sequence with a sequence identity of , at least 70, 75, 80, 85, 90, 91, 92, 93, 94, 95, 96, 97, 98 or 99% to SEQ ID NO: 4 are used in the step (a).
SEQ ID NO: 3
   TTACAAACATTGGCCGCAAA
SEQ ID NO: 4
   GCGCGACATTCCGAAGAA

In another more preferred embodiment of the step (a) of the method of the invention, the forward primer comprises, or consists of, the SEQ ID NO: 3, and the reverse primer comprises, or consists of, the SEQ ID NO: 4.

In another preferred embodiment of the method of the invention a forward primer, which comprises, or consists of, a nucleotide sequence with a sequence identity of, at least 70, 75, 80, 85, 90, 91, 92, 93, 94, 95, 96, 97, 98 or 99% to SEQ ID NO: 5, and a reverse primer which comprises, or consists of, a nucleotide sequence with a sequence identity of , at least 70, 75, 80, 85, 90, 91, 92, 93, 94, 95, 96, 97, 98 or 99% to SEQ ID NO: 6 are used in the step (a).
SEQ ID NO: 5
   ACAGGTACGTTAATAGTTAATAGCGT
SEQ ID NO: 6
   ATATTGCAGCAGTACGCACACA

In another more preferred embodiment of the step (a) of the method of the invention, the forward primer comprises, or consists of, the SEQ ID NO: 5, and the reverse primer comprises, or consists of, the SEQ ID NO: 6.

In another preferred embodiment of the method of the invention a forward primer, which comprises, or consists of, a nucleotide sequence with a sequence identity of, at least 70, 75, 80, 85, 90, 91, 92, 93, 94, 95, 96, 97, 98 or 99% to SEQ ID NO: 7, and a reverse primer which comprises, or consists of, a nucleotide sequence with a sequence identity of , at least 70, 75, 80, 85, 90, 91, 92, 93, 94, 95, 96, 97, 98 or 99% to SEQ ID NO: 8 are used in the step (a).
SEQ ID NO: 7
   GCTGCAATATTGTTAACGTGAG
SEQ ID NO: 8
   GGAACTCCTTCAGAAGAGTTC

In another more preferred embodiment of the step (a) of the method of the invention, the forward primer comprises, or consists of, the SEQ ID NO: 7, and the reverse primer comprises, or consists of, the SEQ ID NO: 8.

In another preferred embodiment of the method of the invention a forward primer, which comprises, or consists of, a nucleotide sequence with a sequence identity of, at least 70, 75, 80, 85, 90, 91, 92, 93, 94, 95, 96, 97, 98 or 99% to SEQ ID NO: 9, and a reverse primer which comprises, or consists of, a nucleotide sequence with a sequence identity of , at least 70, 75, 80, 85, 90, 91, 92, 93, 94, 95, 96, 97, 98 or 99% to SEQ ID NO: 10 are used in the step (a).
SEQ ID NO: 9
   GGCACTGAGGACCCACGTT
SEQ ID NO: 10
   TTGCGACATACCCATAAAAGCA

In another more preferred embodiment of the step (a) of the method of the invention, the forward primer comprises, or consists of, the SEQ ID NO: 9, and the reverse primer comprises, or consists of, the SEQ ID NO: 10.

The term "amplicon", as used herein, refers to a nucleic acid fragment, in the present invention dsDNA, that is the product of amplification or replication events. An amplicon can be artificially generated from a nucleic acid of interest, either RNA or DNA, by an amplification step.

The phrase "at least one amplicon", refers to one or more amplicons, from one or more nucleic acids. A different amplicon is obtained from each different target viral nucleotide sequence, as in the simultaneous detection embodiments of the invention.

In some embodiments of the method of the invention, when the virus detection comprises the simultaneous detection of, at least, 2 viruses and/or when the specific viral sequence detection comprises the simultaneous detection of, at least 2 specific viral sequences, at least 2 amplicons are obtained in the step (a). In a preferred embodiment, 2 amplicons are obtained in the step (a) of the method of the invention. In another preferred embodiment of the method of the invention, 3 amplicons are obtained in the step (a).

In a preferred embodiment of the invention, the amplicon is obtained from a respiratory virus and/or from a biological sample isolated from a subject infected with a respiratory virus.

In another more preferred embodiment of the invention, the amplicon is obtained from a coronavirus and/or from a biological sample isolated from a subject infected with a coronavirus.

In another preferred embodiment of the invention, the amplicon is obtained from SARS-CoV-2 and/or from a biological sample isolated from a subject infected with SARS-CoV-2.

In another preferred embodiment of the invention, the amplicon is obtained from SARS-CoV-1 and/or from a biological sample isolated from a subject infected with SARS-CoV-1.

In another preferred embodiment of the invention, the amplicon is obtained from MERS-CoV and/or from a biological sample isolated from a subject infected with MERS-CoV.

In another preferred embodiment of the invention, the amplicon is obtained from a region of the SARS-CoV-2 E-gene. Preferably, the E-gene region comprises, or consists of, the nucleotide sequence SEQ ID NO: 31
SEQ ID NO: 31

ACAGGUACGUUAAUAGUUAAUAGCGUACUUCUUUUUCUUGCUUUCGUGGUA
UUCUUGCUAGUUACACUAGCCAUCCUUACUGCGCUUCGAUUGUGUGCGUAC
UGCUGCAAUAU

In another preferred embodiment of the invention, the amplicon is obtained from a region of the SARS-CoV-2 N-gene. Preferably, the N-gene region comprises, or consists of, the nucleotide sequence SEQ ID NO: 29 (N1-gene region) or SEQ ID NO: 30 (N2-gene region)
SEQ ID NO: 29 
GACCCCAAAAUCAGCGAAAUGCACCCCGCAUUACGUUUGGUGGACCCUCAGA
UUCAACUGGCAGUAACCAGA
SEQ ID NO: 30

UUACAAACAUUGGCCGCAAAUUGCACAAUUUGCCCCCAGCGCUUCAGCGUUC
UUCGGAAUGUCGCGC

In another preferred embodiment of the invention, the amplicon is obtained from a region of the SARS-CoV-1 E-gene. Preferably, the E-gene region comprises, or consists of, the nucleotide sequence SEQ ID NO: 32.
SEQ ID NO: 32

GCUGCAAUAUUGUUAACGUGAGUUUAGUAAAACCAACGGUUUACGUCUACUC
GCGUGUUAAAAAUCUGAACUCUUCUGAAGGAGUUCC

In another preferred embodiment of the invention, the amplicon is obtained from a region of the MERS-CoV N-gene. Preferably, the N-gene region comprises, or consists of, the nucleotide sequence SEQ ID NO: 33
SEQ ID NO: 33

GGCACUGAGGACCCACGUUGGCCCCAAAUUGCUGAGCUUGCUCCUACAGCC
AGUGCUUUUAUGGGUAUGUCGCAA

In another preferred embodiment of the invention, alone or in combination with the rest of the preferred embodiments of the method of the invention, the amplicon comprises or consists of, a nucleotide sequence with a sequence identity of, at least 70, 75, 80, 85, 90, 91, 92, 93, 94, 95, 96, 97, 98 or 99% to a nucleotide sequence selected from the list of sequences consisting of: SEQ ID NO: 11, SEQ ID NO: 12, SEQ ID NO: 13, SEQ ID NO: 14 and SEQ ID NO: 15.

In a more preferred embodiment of the method of the invention, the amplicon comprises or consists of, a nucleotide sequence selected from the list of sequences consisting of: SEQ ID NO: 11, SEQ ID NO: 12, SEQ ID NO: 13, SEQ ID NO: 14 and SEQ ID NO: 15.
SEQ ID NO: 11 
GACCCCAAAATCAGCGAAATGCACCCCGCATTACGTTTGGTGGACCCTCAGAT
TCAACTGGCAGTAACCAGA
SEQ ID NO: 12 
TTACAAACATTGGCCGCAAATTGCACAATTTGCCCCCAGCGCTTCAGCGTTCTT
CGGAATGTCGCGC
SEQ ID NO: 13 
ACAGGTACGTTAATAGTTAATAGCGTACTTCTTTTTCTTGCTTTCGTGGTATTCT
TGCTAGTTACACTAGCCATCCTTACTGCGCTTCGATTGTGTGCGTACTGCTGCA
ATAT
SEQ ID NO: 14 
GCTGCAATATTGTTAACGTGAGTTTAGTAAAACCAACGGTTTACGTCTACTCGC
GTGTTAAAAATCTGAACTCTTCTGAAGGAGTTCC
SEQ ID NO: 15

GGCACTGAGGACCCACGTTGGCCCCAAATTGCTGAGCTTGCTCCTACAGCCAG
TGCTTTTATGGGTATGTCGCAA

In some preferred embodiments of the method of the invention, in particular, in simultaneous detection preferred embodiments, alone or in combination with other preferred embodiments of the method of the invention, the amplicons comprise or consist of, nucleotide sequences with sequence identity of, at least 70, 75, 80, 85, 90, 91, 92, 93, 94, 95, 96, 97, 98 or 99% to nucleotide sequences selected from the list of sequences consisting of: SEQ ID NO: 11, SEQ ID NO: 12, SEQ ID NO: 13, SEQ ID NO: 14, SEQ ID NO: 15, and any combination thereof.

In some more preferred embodiments of the method of the invention, particularly, in simultaneous detection preferred embodiments, alone or in combination with other preferred embodiments of the method of the invention, the amplicons nucleotide sequences are selected from the list of sequences consisting of: SEQ ID NO: 11, SEQ ID NO: 12, SEQ ID NO: 13, SEQ ID NO: 14, SEQ ID NO: 15, and any combination thereof.

The term "protospacer adjacent motif (PAM)", as used herein, refers to a short DNA sequence (usually 2-6 base pairs in length) located next to the DNA targeted region by a CRISPR system, such as CRISPR-Cas9. Cas proteins, such as Cas9, recognize and bind the PAM sequence and unwind the adjacent dsDNA helix. The opened DNA at this local point allows initiating the hybridization between the sgRNA and the targeted DNA strand, producing a branch migration process ending with the formation of the final R-loop structure, in which the spacer of the sgRNA is totally paired to the targeted strand and the non-targeted strand is displaced but still bound to the Cas9.

Cas nucleases isolated from different bacterial species recognize different PAM sequences. For instance, the SpCas9 (Cas9 protein from the species *Streptococcus pyogenes)* nuclease cuts upstream of the PAM sequence 5'-NGG-3' (where "N" can be any nucleotide base). Thus, in a preferred embodiment, the PAM sequence comprises 5'-NGG-3', wherein N is any nucleobase.

A PAM may be introduced into the amplicon using specially designed primers for amplification of the target viral nucleotide sequence to form an amplicon comprising a PAM. The PAM may include a PAM from *Neisseria meningitidis, Treponema denticola, Streptococcus thermophilus, Streptococcus pyogenes, Staphylococcus aureus, Francisella novicida*, or *Campylobacter jejuni*, or a variant thereof, or a combination thereof. Preferably, the PAM is from *Streptococcus pyogenes.*

As used herein, a "target viral nucleotide sequence" refers to a nucleotide sequence of interest of a viral nucleic acid, including ssDNA, dsDNA, ssRNA, or dsRNA, which is amplified as it has been previously disclosed to obtain an amplicon from it.

The phrase "at least one target viral nucleotide sequence", refers to one or more target viral nucleotide sequences of one or more viral nucleic acids. In some embodiments of the method of the invention, when the virus detection comprises the simultaneous detection of at least 2 viruses and/or when the specific viral sequence detection comprises the simultaneous detection of at least 2 specific viral sequences, the step (a) of the method of the invention comprises amplifying at least 2 target viral nucleotide sequences from a sample. In a preferred embodiment, the step (a) of the method of the invention comprises amplifying 2 target viral nucleotide sequences. In another preferred embodiment, the step (a) of the method of the invention comprises amplifying 3 target viral nucleotide sequences.

In a preferred embodiment of the method of the invention, the target viral nucleotide sequence is from, at least, one respiratory virus.

In the present invention, a "respiratory virus" refers to a virus which is transmitted via the respiratory route and which may cause both, upper respiratory tract symptoms and/or lower respiratory tract symptoms. Examples of respiratory viruses include, without limitation to, coronavirus, influenza A virus, influenza B virus, adenovirus, human bocavirus (HBoV), human metapneumovirus (HMPV), parainfluenza virus, respiratory syncytial virus (RSV), rhinovirus and enterovirus.

Thus, in a preferred embodiment of the method of the invention, the respiratory virus is selected from the list consisting of: coronavirus, influenza A virus, influenza B virus, adenovirus, human bocavirus (HBoV), human metapneumovirus (HMPV), parainfluenza virus, respiratory syncytial virus (RSV), rhinovirus, enterovirus, and any combination thereof.

Preferably, the respiratory virus is a coronavirus. Examples of coronaviruses include, without limitation to, severe acute respiratory syndrome coronavirus 2 (SARS-CoV-2), severe acute respiratory syndrome coronavirus (SARS-CoV-1), middle east respiratory syndrome coronavirus (MERS-CoV), human coronavirus 229E, human coronavirus NL63, Miniopterus bat coronavirus 1 ,Miniopterus bat coronavirus HKU8, porcine epidemic diarrhea virus, Rhinolophus bat coronavirus HKU2, Scotophilus bat coronavirus 512, bovine coronavirus, human coronavirus OC43, human coronavirus HKU1, murine coronavirus, Pipistrellus bat coronavirus HKU5, Rousettus bat coronavirus HKU9, Tylonycteris bat coronavirus HKU4, hedgehog coronavirus 1, infectious bronchitis virus, beluga whale coronavirus SW1, infectious bronchitis virus, Bulbul coronavirus HKU11, pangolin coronavirus, porcine coronavirus HKU15, WIVI-CoV, SHC014-CoV, bat-SL-CoVZC45, bat-SLCoVZXC21, SARS-CoVGZ02, BtKY72, WIV16, Rs4231, Rs7327, Rs9401, BtRs- BetaCoV/YN2018R, BtRs-BetaCoV/YN2013, Anlong-112, Rf2092, BtRs- BetaCoV/YN2018C, As6526, Rs4247, BtRs-BetaCoV/GX2013, Yunnan2011, BtRI- BetaCoV/SC2018, Shannxi2011, BtRs-BetaCoV/HuB2013, Bat_CoV_279/2005, HKU3-12, HKU3-3, HKU3-7, Longquan-140, RaTG13 and a variant thereof.

In a more preferred embodiment of the method of the invention, the coronavirus is selected from the list consisting of: severe acute respiratory syndrome coronavirus 2 (SARS-CoV-2), severe acute respiratory syndrome coronavirus (SARS-CoV-1), middle east respiratory syndrome coronavirus (MERS-CoV), human coronavirus 229E, human coronavirus NL63, Miniopterus bat coronavirus 1 ,Miniopterus bat coronavirus HKU8, porcine epidemic diarrhea virus, Rhinolophus bat coronavirus HKU2, Scotophilus bat coronavirus 512, bovine coronavirus, human coronavirus OC43, human coronavirus HKU1, murine coronavirus, Pipistrellus bat coronavirus HKU5, Rousettus bat coronavirus HKU9, Tylonycteris bat coronavirus HKU4, hedgehog coronavirus 1, infectious bronchitis virus, beluga whale coronavirus SW1, infectious bronchitis virus, Bulbul coronavirus HKU11, pangolin coronavirus, porcine coronavirus HKU15, WIVI-CoV, SHC014-CoV, bat-SL-CoVZC45, bat-SLCoVZXC21, SARS-CoVGZ02, BtKY72, WIV16, Rs4231, Rs7327, Rs9401, BtRs-BetaCoV/YN2018R, BtRs-BetaCoV/YN2013, Anlong-112, Rf2092, BtRs-BetaCoV/YN2018C, As6526, Rs4247, BtRs-BetaCoV/GX2013, Yunnan2011, BtRI-BetaCoV/SC2018, Shannxi2011, BtRs-BetaCoV/HuB2013, Bat_CoV_279/2005, HKU3-12, HKU3-3, HKU3-7, Longquan-140, RaTG13, and any combination thereof..

More preferably, the coronavirus is selected from the list consisting of SARS-CoV-2, SARS-CoV-1, MERS-CoV, and any combination thereof

In an even more preferred embodiment of the method of the invention, when the virus is SARS-CoV-2, the target viral nucleotide sequences comprise, or consist of, nucleotide sequence SEQ ID NO: 29, SEQ ID NO: 30, SEQ ID NO: 31, and/or any combination thereof.

In another even more preferred embodiment of the method of the invention, when the virus is SARS-CoV-1, the target viral nucleotide sequence comprises, or consists of, nucleotide sequence SEQ ID NO: 32.

In another even more preferred embodiment of the method of the invention, when the virus is MERS-CoV, the target viral nucleotide sequence comprises, or consists of, nucleotide sequence SEQ ID NO: 33.

In some preferred embodiment of the method of the invention, alone or in combination with the rest of preferred embodiments, the target viral nucleotide sequence/s comprise, or consist of, nucleotide sequences with sequence identity of, at least 70, 75, 80, 85, 90, 91, 92, 93, 94, 95, 96, 97, 98 or 99% to nucleotide sequences selected from the list of sequences consisting of SEQ ID NO: 29, SEQ ID NO: 30, SEQ ID NO: 31, SEQ ID NO: 32, SEQ ID NO: 33 and any combination thereof.

In some more preferred embodiments of the method of the invention, alone or in combination with other preferred embodiments of the method of the invention, the target viral nucleotide sequence/s are selected from the list of sequences consisting of: SEQ ID NO: 29, SEQ ID NO: 30, SEQ ID NO: 31, SEQ ID NO: 32, SEQ ID NO: 33, and any combination thereof.

In the present invention, the term "sample" refers to a part or small quantity of a thing which is considered representative of the whole and which is taken or separated from it for the purpose of study, analysis or experimentation. In a preferred embodiment of the method of the invention, the sample is a previously isolated biological sample.

A "biological sample", as used herein, refers to a sample isolated from a subject. The biological sample can be isolated from a subject infected or not with a virus. The term "subject", as used in the present document, refers to any animal, preferably a mammal, and includes, but is not limited to, domestic and farm animals, primates, and humans. In a preferred embodiment, the subject is a human being.

Biological samples include, without limitation to, mucous samples, faecal samples, and blood samples. Preferably, the biological sample is a mucous sample. More preferably, the biological sample is a mucous sample obtained from nasopharyngeal swab, oropharyngeal swab/sampling, nasal swab, and/or buccal swab.

In another preferred embodiment, the isolated biological sample is from a subject infected with, at least, one virus, more preferably, from a subject infected with, at least, one respiratory virus. The term "respiratory virus", as well as all its preferred embodiments, have been previously described and apply equally hereinafter. In an even more preferred embodiment, the isolated biological sample is from a subject infected with at least one respiratory virus selected from the list consisting of SARS-CoV-2, SARS-CoV-1, MERS-CoV, and any combination thereof.

In a preferred embodiment, the subject is a subject infected with, at least, one virus. Preferably, the subject is a patient infected with, at least, one respiratory virus.

More preferably, the subject is a patient infected with, at least, one respiratory virus selected from the list consisting of SARS-CoV-2, SARS-CoV-1, MERS-CoV, and any combination thereof.

### Step (b): contacting the amplicon to a Cas9 and a sgRNA

A second step of the method of the invention [step (b)], comprises contacting the amplicon/s obtained in the step (a) to a Cas9 protein and, at least, one sgRNA, wherein the sgRNA comprises a nucleotide sequence that hybridizes with a target region of the amplicon obtained in (a), obtaining at least one ribonucleoprotein-DNA complex.

### Contacting the amplicon

The term "contacting", as used herein, refers to put in contact the amplicon/s obtained in the step (a) with the sgRNA/s and the Cas9 protein in a reaction mixture. In the present invention, when the amplicon is contacted to Cas9 and a sgRNA, a ribonucleoprotein-DNA complex is formed.

The term "ribonucleoprotein-DNA complex", as used in the present invention, refers to a molecular complex comprising the amplicon, the sgRNA and the Cas9 protein. The phrase "at least one ribonucleoprotein-DNA complex", refers to one or more ribonucleoprotein-DNA complexes obtained in the step (b). In the method of the invention, each different virus and/or specific viral sequence to be detected in the sample, generates a different ribonucleoprotein-DNA complex, each one of which comprising a different amplicon of DNA and a different sgRNA, and a Cas9 protein.

In a preferred embodiment, the amplicon concentration is from 10 to 400 nM (including the end values of the range). More preferably, the amplicon concentration is from 10 to 80 nM (including the end values of the range). Even more preferably, the amplicon concentration is 10, 20, 30, 40, 50, 60, 70 or 80 nM.

In some embodiments of the method of the invention, the Cas9 protein and the sgRNA/s may be allowed to form a Cas9:sgRNA complex prior to the addition to the reaction mixture with the amplicon/s. Thus, in a preferred embodiment, alone or in combination with the resto of the embodiments, the method of the invention comprises a further step [step (a')], after the step (a)), which comprises assembling the Cas9 and sgRNA, previously to be contacted with the amplicon/s obtained in the step (a). More preferably, this step (a') of assembling the Cas9 and sgRNA, comprises mixing the Cas9 protein and sgRNA during at least 30 minutes, at a temperature of 15 to 30 ºC (including the end values of the range), even more preferably, at a temperature of 25ºC. Preferably, the proportion of Cas9 and the sgRNA in this step (a') is 1:1.

In a still more preferred embodiment of the step (a'), the concentration of the Cas9:sgRNA complex assembled is from 50 nM to 200 nM (including the end values of the range). Even more preferably, the concentration of the Cas9:sgRNA complex assembled is 100 nM.

In the method of the invention, the sgRNA/s, the Cas9 protein and the amplicon/s may be added to the reaction mixture sequentially or at the same time.

Thus, in another preferred embodiment of the method of the invention, the sgRNA/s, the Cas9 protein and the amplicon/s are added to the reaction mixture at the same time.

In another preferred embodiment of the method of the invention, the sgRNA/s, the Cas9 protein and the amplicon/s are added to the reaction mixture sequentially.

Furthermore, as it is known by a person skilled in the art, a reaction mixture can include other reagents and/or buffers. Examples of buffer and reagents that may be used in the step (b) of the method of the invention include, without limitation to, TAE buffer (Tris-Acetate-EDT buffer) supplemented with MgCl₂ and Tween 20, or Tris hydrochloride buffers, preferably at a basic pH (e.g., pH 8), supplemented with MgCl₂, or similar.

In a preferred embodiment, the step (b), comprising contacting the amplicon/s with the sgRNA/s and the Cas9 protein, is performed under certain reaction conditions. Preferably, said reaction conditions comprise the incubation of the amplicon/s with the sgRNA/s and the Cas9 protein at a temperature of 25 ºC to 40 ºC (including the end values of the range), for 10 to 30 min (including the end values of the range).More preferably, said reaction conditions comprise the incubation of the amplicon/s with the sgRNA/s and the Cas9 protein at 37ºC for 20 min. Even more preferably, the incubation is carried out in a thermocycler, thermomixer, thermoblock, or similar.

### Cas9 protein and sgRNA

The term "Cas9" refers to an RNA-guided protein comprising a Cas9 protein, or a fragment thereof. Cas9 is also referred to a CRISPR (clustered regularly interspaced short palindromic repeat)-associated nuclease. CRISPR is an adaptive immune system that provides protection against mobile genetic elements (e.g., viruses, transposable elements and conjugative plasmids). CRISPR clusters contain spacers, sequences complementary to antecedent mobile elements, and target invading nucleic acids.

CRISPR clusters are transcribed and processed into CRISPR RNA (crRNA). In type II CRISPR systems correct processing of pre-crRNA requires a trans-encoded small RNA (tracrRNA), endogenous ribonuclease 3 (rnc) and a Cas9 protein.

However, single guide RNAs ("sgRNA", or simply "gRNA") can be engineered to incorporate aspects of both the crRNA and tracrRNA into a single RNA molecule (See, e.g., Jinek M., Chylinski K., Fonfara I., Hauer M., Doudna J.A., Charpentier E. Science 337:816-821(2012)). Cas9 recognizes a short motif in the CRISPR repeat sequences (the PAM or protospacer adjacent motif) to help distinguish self versus non-self.

In the present invention, the Cas9 protein may be any suitable Cas9 protein which comprises strand displacement activity, and which is able to form a complex with a target nucleotide sequence or amplicon, in conjunction with a sgRNA.

In a preferred embodiment of the method of the invention, the Cas9 protein does not comprise collateral catalytic activity. The term "collateral catalytic activity", as used herein, refers to non-specific oligonucleotide cleaving activity displayed by some Cas proteins (such as Cas12 and Cas13).

Cas9 nuclease sequences and structures are well known to those of skill in the art (see, e.g., "Complete genome sequence of an MI strain of Streptococcus pyogenes." Ferretti J.J., McShan W.M., Ajdic D.J., Savic D.J., Savic G., Lyon K., Primeaux C, Sezate S., Suvorov A.N., Kenton S., Lai H.S., Lin S.P., Qian Y., Jia H.G., Najar F.Z., Ren Q., Zhu H., Song L. expand/collapse author list McLaughlin R.E., Proc. Natl. Acad. Sci. U.S.A. 98:4658-4663(2001); "CRISPR RNA maturation by trans-encoded small RNA and host factor RNase III." Deltcheva E., Chylinski K., Sharma CM., Gonzales K., Chao Y., Pirzada Z.A., Eckert M.R., Vogel J., Charpentier E., Nature 471:602-607(2011); and "A programmable dual-RNA-guided DNA endonuclease in adaptive bacterial immunity." Jinek M., Chylinski K., Fonfara I., Hauer M., Doudna J.A., Charpentier E. Science 337:816-821(2012)).

Cas9 orthologs have been described in various species, including, but not limited to, *S*. *pyogenes* and *S*. *thermophilus.* Cas9 proteins are known to exist in many Type II CRISPR systems including the following as identified in the supplementary information to Makarova et al., Nature Reviews, Microbiology, Vol. 9, June 2011, pp. 467-477).

In a preferred embodiment of the method of the invention, alone or in combination with the rest of the embodiments, the Cas9 protein is from the species *Streptococcus pyogenes* (SpCas9). More preferably, the *Streptococcus pyogenes* Cas9 is selected from the list consisting of: wild type Cas9 nuclease, Cas9 H840A nickase and catalytically dead Cas9.

"Wild type Cas9 nuclease", is a Cas9 protein from *Streptococcus pyogenes.* In a preferred embodiment of the method of the invention, the wild type Cas9 nuclease comprises, or consists of, an amino acid sequence with a sequence identity of, at least, 70, 75, 80, 85, 90, 91, 92, 93, 94, 95, 96, 97, 98 or 99% to sequence SEQ ID NO: 16.
SEQ ID NO: 16:

The sequence SEQ ID NO: 16, corresponds to the *Streptococcus pyogenes* wild type Cas9 amino acid sequence. In a more preferred embodiment of the method of the invention, wild type Cas9 nuclease comprises, or consists of, the amino acid sequence SEQ ID NO: 16.

"Cas9 H840A nickase" (or Cas9n) refers to a partially catalytically inactive form of Cas9 working like a nickase that has the H840A mutation (in the HNH domain), and which only cleaves the non-targeted strand of the amplicon nucleotide sequence. In a preferred embodiment of the method of the invention, the Cas9 H840A nickase comprises, or consists of, an amino acid sequence with a sequence identity of, at least, 70, 75, 80, 85, 90, 91, 92, 93, 94, 95, 96, 97, 98 or 99% with the sequence SEQ ID NO: 17.
SEQ ID NO: 17:

MDKKYSIGLDIGTNSVGWAVITDEYKVPSKKFKVLGNTDRHSIKKNLIGALLFDSGE
TAEATRLKRTARRRYTRRKNRICYLQEIFSNEMAKVDDSFFHRLEESFLVEEDKKH
ERHPIFGNIVDEVAYHEKYPTIYHLRKKLVDSTDKADLRLIYLALAHMIKFRGHFLIEG
DLNPDNSDVDKLFIQLVQTYNQLFEENPINASGVDAKAILSARLSKSRRLENLIAQLP
GEKKNGLFGNLIALSLGLTPNFKSNFDLAEDAKLQLSKDTYDDDLDNLLAQIGDQYA
DLFLAAKNLSDAILLSDILRVNTEITKAPLSASMIKRYDEHHQDLTLLKALVRQQLPE
KYKEIFFDQSKNGYAGYIDGGASQEEFYKFIKPILEKMDGTEELLVKLNREDLLRKQ
RTFDNGSIPHQIHLGELHAILRRQEDFYPFLKDNREKIEKILTFRIPYYVGPLARGNS
RFAWMTRKSEETITPWNFEEVVDKGASAQSFIERMTNFDKNLPNEKVLPKHSLLYE
YFTVYNELTKVKYVTEGMRKPAFLSGEQKKAIVDLLFKTNRKVTVKQLKEDYFKKIE
CFDSVEISGVEDRFNASLGTYHDLLKIIKDKDFLDNEENEDILEDIVLTLTLFEDREMI
EERLKTYAHLFDDKVMKQLKRRRYTGWGRLSRKLINGIRDKQSGKTILDFLKSDGF
ANRNFMQLIHDDSLTFKEDIQKAQVSGQGDSLHEHIANLAGSPAIKKGILQTVKVVD

ELVKVMGRHKPENIVIEMARENQTTQKGQKNSRERMKRIEEGIKELGSQILKEHPV
ENTQLQNEKLYLYYLQNGRDMYVDQELDINRLSDYDVDAIVPQSFLKDDSIDNKVL
TRSDKNRGKSDNVPSEEVVKKMKNYWRQLLNAKLITQRKFDNLTKAERGGLSELD
KAGFIKRQLVETRQITKHVAQILDSRMNTKYDENDKLIREVKVITLKSKLVSDFRKDF
QFYKVREINNYHHAHDAYLNAVVGTALIKKYPKLESEFVYGDYKVYDVRKMIAKSE
QEIGKATAKYFFYSNIMNFFKTEITLANGEIRKRPLIETNGETGEIVWDKGRDFATVR
KVLSMPQVNIVKKTEVQTGGFSKESILPKRNSDKLIARKKDWDPKKYGGFDSPTVA
YSVLVVAKVEKGKSKKLKSVKELLGITIMERSSFEKNPIDFLEAKGYKEVKKDLIIKLP
KYSLFELENGRKRMLASAGELQKGNELALPSKYVNFLYLASHYEKLKGSPEDNEQ
KQLFVEQHKHYLDEIIEQISEFSKRVILADANLDKVLSAYNKHRDKPIREQAENIIHLF
TLTNLGAPAAFKYFDTTIDRKRYTSTKEVLDATLIHQSITGLYETRIDLSQLGGD

The sequence SEQ ID NO: 17, corresponds to the *Streptococcus pyogenes* Cas9 H840A nickase amino acid sequence. In a more preferred embodiment of the method of the invention, Cas9 H840A nickase comprises, or consists of, the amino acid sequence SEQ ID NO: 17.

"Catalytically dead Cas9" (or dCas9) refers to a catalytically dead Cas9 protein that lacks endonuclease activity, and which does not produce any cleavage. In a preferred embodiment of the method of the invention, the catalytically dead Cas9 comprises, or consists of, an amino acid sequence with a sequence identity of, at least, 70, 75, 80, 85, 90, 91, 92, 93, 94, 95, 96, 97, 98 or 99% with the sequence SEQ ID NO: 18.
SEQ ID NO: 18:

MDKKYSIGLAIGTNSVGWAVITDEYKVPSKKFKVLGNTDRHSIKKNLIGALLFDSGE
TAEATRLKRTARRRYTRRKNRICYLQEIFSNEMAKVDDSFFHRLEESFLVEEDKKH
ERHPIFGNIVDEVAYHEKYPTIYHLRKKLVDSTDKADLRLIYLALAHMIKFRGHFLIEG
DLNPDNSDVDKLFIQLVQTYNQLFEENPINASGVDAKAILSARLSKSRRLENLIAQLP
GEKKNGLFGNLIALSLGLTPNFKSNFDLAEDAKLQLSKDTYDDDLDNLLAQIGDQYA
DLFLAAKNLSDAILLSDILRVNTEITKAPLSASMIKRYDEHHQDLTLLKALVRQQLPE
KYKEIFFDQSKNGYAGYIDGGASQEEFYKFIKPILEKMDGTEELLVKLNREDLLRKQ
RTFDNGSIPHQIHLGELHAILRRQEDFYPFLKDNREKIEKILTFRIPYYVGPLARGNS
RFAWMTRKSEETITPWNFEEVVDKGASAQSFIERMTNFDKNLPNEKVLPKHSLLYE
YFTVYNELTKVKYVTEGMRKPAFLSGEQKKAIVDLLFKTNRKVTVKQLKEDYFKKIE

CFDSVEISGVEDRFNASLGTYHDLLKIIKDKDFLDNEENEDILEDIVLTLTLFEDREMI
EERLKTYAHLFDDKVMKQLKRRRYTGWGRLSRKLINGIRDKQSGKTILDFLKSDGF
ANRNFMQLIHDDSLTFKEDIQKAQVSGQGDSLHEHIANLAGSPAIKKGILQTVKVVD
ELVKVMGRHKPENIVIEMARENQTTQKGQKNSRERMKRIEEGIKELGSQILKEHPV
ENTQLQNEKLYLYYLQNGRDMYVDQELDINRLSDYDVDAIVPQSFLKDDSIDNKVL
TRSDKNRGKSDNVPSEEVVKKMKNYWRQLLNAKLITQRKFDNLTKAERGGLSELD
KAGFIKRQLVETRQITKHVAQILDSRMNTKYDENDKLIREVKVITLKSKLVSDFRKDF
QFYKVREINNYHHAHDAYLNAVVGTALIKKYPKLESEFVYGDYKVYDVRKMIAKSE
QEIGKATAKYFFYSNIMNFFKTEITLANGEIRKRPLIETNGETGEIVWDKGRDFATVR
KVLSMPQVNIVKKTEVQTGGFSKESILPKRNSDKLIARKKDWDPKKYGGFDSPTVA
YSVLVVAKVEKGKSKKLKSVKELLGITIMERSSFEKNPIDFLEAKGYKEVKKDLIIKLP
KYSLFELENGRKRMLASAGELQKGNELALPSKYVNFLYLASHYEKLKGSPEDNEQ
KQLFVEQHKHYLDEIIEQISEFSKRVILADANLDKVLSAYNKHRDKPIREQAENIIHLF
TLTNLGAPAAFKYFDTTIDRKRYTSTKEVLDATLIHQSITGLYETRIDLSQLGGD

The sequence SEQ ID NO: 18, corresponds to the *Streptococcus pyogenes* catalytically dead Cas9 amino acid sequence. In a more preferred embodiment of the method of the invention, catalytically dead Cas9 comprises, or consists of, the amino acid sequence SEQ ID NO: 18.

Single guide (sgRNA) can also be referred to as guide RNAs (gRNA), terms interchangeably used in the present document. The sgRNA may be any suitable sgRNA that enables the Cas9 protein to form a complex with the target nucleotide sequence or amplicon region.

The phrase "at least one sgRNA", refers to one or more sgRNA, depending on the number of viruses and/or specific viral sequences to be detected in the sample. Thus, a different sgRNA (each different sgRNA comprises a different nucleotide sequence to hybridize with each different target amplicon region) is used in the method of the invention for each different virus and/or specific viral sequence detection, as in the simultaneous detection embodiments of the invention.

Thus, in some embodiments of the method of the invention, when the virus detection comprises the simultaneous detection of at least 2 viruses and/or when the specific viral sequence detection comprises the simultaneous detection of at least 2 specific viral sequences, the step (b) of the method of the invention comprises contacting the amplicon/s obtained in the step (a) to a Cas9 protein and at least 2 sgRNAs. A preferred embodiment of the step (b) comprises contacting the amplicon/s obtained in the step (a) to a Cas9 protein and 2 sgRNAs. In another preferred embodiment, the step (b) comprises contacting the amplicon/s obtained in the step (a) to a Cas9 protein and 3 sgRNAs.

As it has been mentioned, in the present invention the sgRNA comprises a nucleotide sequence that hybridizes with a target region of the amplicon obtained in (a).

As used herein, the term "target region of the amplicon", refers to a nucleotide sequence present in the amplicon that is recognised and to which the sgRNA hybridizes.

The terms "hybridize" or "hybridization", as used in the present invention, refers to interactions based on base-pairing between two nucleotide sequences.

In the present invention, the nucleotide sequence that hybridizes with the target amplicon region (also called as "spacer" of the sgRNA) may harbour a mutation in the PAM-distal region to form a wobble base pair with the targeted nucleotide sequence or amplicon region, but not with the molecular beacon.

Thus, in a preferred embodiment of the method of the invention, the sgRNA spacer comprises a nucleotide sequence which does not hybridize spontaneously to the molecular beacon arranged in a hairpin structure (closed-form of the molecular beacon). More preferably, the sequence of the sgRNA which does not hybridize to the molecular beacon arranged in a hairpin structure is the part of the spacer that targets the PAM-proximal region.

In a preferred embodiment of the invention, alone or in combination with the rest of preferred embodiments of the invention, the sgRNA comprises a nucleotide sequence that hybridizes with a target region of an amplicon obtained from a respiratory virus and/or from a biological sample isolated from a subject infected with a respiratory virus.

In another more preferred embodiment of the invention, alone or in combination with the rest of preferred embodiments of the invention, the sgRNA comprises a nucleotide sequence that hybridizes with an amplicon obtained from a coronavirus and/or from a biological sample isolated from a subject infected with a coronavirus.

In another preferred embodiment of the invention, alone or in combination with the rest of preferred embodiments, the sgRNA comprises a nucleotide sequence that hybridizes with target region of the amplicon obtained from SARS-CoV-2 and/or from a biological sample isolated from a subject infected with SARS-CoV-2.

In a more preferred embodiment of the method of the invention alone or in combination with the rest of preferred embodiments of the invention, the sgRNA comprises, or consists of, a nucleotide sequence with a sequence identity of, at least, 70, 75, 80, 85, 90, 91, 92, 93, 94, 95, 96, 97, 98 or 99% to sequence SEQ ID NO: 19, SEQ ID NO: 20 or SEQ ID NO: 21
SEQ ID NO: 19: 
GGAAAAUUAGCGAAAUGCACCCCGCAUUACGUUGUUUUAGAGCUAGAAAUAG
CAAGUUAAAAUAAGGCUAGUCCGUUAUCAACUUGAAAAAGUGGCACCGAGUC
GGUGCUUUU
SEQ ID NO: 20: 
GGCAUUUCGAAGAACGCUGAAGCGCGUUUUAGAGCUAGAAAUAGCAAGUUAA
AAUAAGGCUAGUCCGUUAUCAACUUGAAAAAGUGGCACCGAGUCGGUGCUU
UU
SEQ ID NO: 21:

GGAGUAGUACGCACACAAUCGAAGCGCAGUAGUUUUAGAGCUAGAAAUAGCA
AGUUAAAAUAAGGCUAGUCCGUUAUCAACUUGAAAAAGUGGCACCGAGUCGG
UGCUUUU

The sequences SEQ ID NO: 19, SEQ ID NO: 20 and SEQ ID NO: 21 correspond to nucleotide sequences able to hybridize with a target region of the amplicon obtained from SARS-CoV-2 and/or from a biological sample isolated from a subject infected with SARS-CoV-2.

In a still more preferred embodiment of the method of the invention, the sgRNA comprises or consists of, the sequence SEQ ID NO: 19, SEQ ID NO: 20 and/or SEQ ID NO: 21.

In another preferred embodiment of the invention, alone or in combination with the rest of preferred embodiments, the sgRNA comprises a nucleotide sequence that hybridizes with target region of the amplicon obtained from SARS-CoV-1 and/or from a biological sample isolated from a subject infected with SARS-CoV-1.

In a more preferred embodiment of the method of the invention, the sgRNA comprises, or consists of, a nucleotide sequence with a sequence identity of, at least, 70, 75, 80, 85, 90, 91, 92, 93, 94, 95, 96, 97, 98 or 99% with the sequence SEQ ID NO: 22
SEQ ID NO: 22

GGAUAUUGUUAAUGUGAGUUUAGUAAAACCAAGUUUUAGAGCUAGAAAUAGC
AAGUUAAAAUAAGGCUAGUCCGUUAUCAACUUGAAAAAGUGGCACCGAGUCG
GUGCUUUU

The sequence SEQ ID NO: 22 corresponds to a nucleotide sequence able to hybridize with a target region of the amplicon obtained from SARS-CoV-1 and/or from a biological sample isolated from a subject infected with SARS-CoV-1. In a still more preferred embodiment of the method of the invention, the sgRNA comprises or consists of, the sequence SEQ ID NO: 22.

In another preferred embodiment of the invention, alone or in combination with the rest of preferred embodiments, the sgRNA comprises a nucleotide sequence that hybridizes with an amplicon obtained from MERS-CoV and/or from a biological sample isolated from a subject infected with MERS-CoV.

In a more preferred embodiment of the method of the invention, the sgRNA comprises, or consists of, a nucleotide sequence with a sequence identity of, at least, 70, 75, 80, 85, 90, 91, 92, 93, 94, 95, 96, 97, 98 or 99% with the sequence SEQ ID NO: 23
SEQ ID NO: 23

GGCAUAUCCAUAAAAGCACUGGCUGUGUUUUAGAGCUAGAAAUAGCAAGUUA
AAAUAAGGCUAGUCCGUUAUCAACUUGAAAAAGUGGCACCGAGUCGGUGCU
UUU

The sequence SEQ ID NO: 23 corresponds to a nucleotide sequence able to hybridize with an amplicon obtained from MERS-CoV and/or from a biological sample isolated from a subject infected with MERS-CoV. In a still more preferred embodiment of the method of the invention, the sgRNA comprises or consists of, the sequence SEQ ID NO: 23.

In another preferred embodiment of the invention, alone or in combination with the rest of preferred embodiments, the sgRNA comprises a nucleotide sequence that hybridizes with a target region of the amplicon obtained from a region of the SARS-CoV-2 E-gene.

In a more preferred embodiment, the sgRNA nucleotide sequence that hybridizes with a target region of the amplicon obtained from a region of the SARS-CoV-2 E-gene comprises, or consists of, a nucleotide sequence with a sequence identity of, at least, 70, 75, 80, 85, 90, 91, 92, 93, 94, 95, 96, 97, 98 or 99% with the sequence SEQ ID NO: 21.

The sequence SEQ ID NO: 21, previously mentioned, corresponds to a nucleotide sequence able to hybridize with a target region of an amplicon obtained from a region of the SARS-CoV-2 E-gene. In a still more preferred embodiment of the method of the invention, the sgRNA nucleotide sequence that hybridizes with a target region of the amplicon obtained from a region of the SARS-CoV-2 E-gene comprises or consists of, the sequence SEQ ID NO: 21.

In another preferred embodiment of the invention alone or in combination with the rest of preferred embodiments, the sgRNA comprises a nucleotide sequence that hybridizes with target region of the amplicon obtained from a region of the SARS-CoV-2 N-gene.

In a more preferred embodiment of the method of the invention, the sgRNA nucleotide sequence that hybridizes with a target region of the amplicon obtained from a region of the SARS-CoV-2 N-gene comprises, or consists of, a nucleotide sequence with a sequence identity of, at least, 70, 75, 80, 85, 90, 91, 92, 93, 94, 95, 96, 97, 98 or 99% with sequence SEQ ID NO: 19 or with sequence SEQ ID NO: 20.

The sequence SEQ ID NO: 19, previously mentioned, corresponds to a nucleotide sequence able to hybridize with a target region of an amplicon obtained from a region of the SARS-CoV-2 N-gene (N1 region). In a still more preferred embodiment of the method of the invention, the sgRNA nucleotide sequence that hybridizes with a target region of the amplicon obtained from a region of the SARS-CoV-2 N-gene (N1 region) comprises or consists of, the sequence SEQ ID NO: 19.

The sequence SEQ ID NO: 20, previously mentioned, corresponds to a nucleotide sequence able to hybridize with a target region of an amplicon obtained from a region of the SARS-CoV-2 N-gene (N2 region). In a still more preferred embodiment of the method of the invention, the sgRNA nucleotide sequence that hybridizes with a target region of the amplicon obtained from a region of the SARS-CoV-2 N-gene (N2 region) comprises or consists of, the sequence SEQ ID NO: 20.

In some preferred embodiments of the method of the invention, in particular, in simultaneous detection preferred embodiments, alone or in combination with other preferred embodiments of the method of the invention, the sgRNAs comprise or consist of, nucleotide sequences with sequence identity of, at least 70, 75, 80, 85, 90, 91, 92, 93, 94, 95, 96, 97, 98 or 99% to nucleotide sequences selected from the list of sequences consisting of: SEQ ID NO: 19, SEQ ID NO: 20 and SEQ ID NO: 21, SEQ ID NO: 22, SEQ ID NO: 23, and any combination thereof.

In some more preferred embodiments of the method of the invention, particularly in simultaneous detection preferred embodiments, alone or in combination with other preferred embodiments of the method of the invention, the sgRNAs nucleotide sequences are selected from the list of sequences consisting of: SEQ ID NO: 19, SEQ ID NO: 20 and SEQ ID NO: 21, SEQ ID NO: 22, SEQ ID NO: 23, and any combination thereof.

In the present invention, "sequence identity" means the degree of similarity between two nucleotide (or amino acid) sequences obtained by aligning the two sequences. Depending on the number of common residues between the aligned sequences, a degree of identity expressed as a percentage will be obtained. The degree of identity between two nucleotide (or amino acid) sequences can be determined by conventional methods, e.g. by standard sequence alignment algorithms known in the state of the art, such as BLAST [Altschul S.F. et al. Basic local alignment search tool. J Mol Biol. 1990 Oct 5; 215(3):403-10]. BLAST programs, e.g. BLASTN, BLASTX, and TBLASTX, BLASTP and TBLASTN, are in the public domain on the website of The National Center for Biotechonology Information (NCBI).

### Step (c): adding a molecular beacon to the ribonucleoprotein-DNA complex obtained in step (b)

A third step of the method of the invention [step (c)], comprises adding, at least, one molecular beacon to the ribonucleoprotein-DNA complex obtained in (b), wherein the molecular beacon comprises a nucleotide sequence that hybridizes with a strand-displaced region of the amplicon, a fluorophore linked to the 3' or 5' end of its nucleotide sequence, and a quencher linked to the remaining end of its nucleotide sequence, arranged in a conformation, such that:
(i) when the molecular beacon does not hybridize to the strand-displaced region of the amplicon, the molecular beacon is arranged in a hairpin structure, with the quencher and fluorophore located spatially close without emitting fluorescence or emitting a basal fluorescence;
(ii) when the molecular beacon hybridizes to the strand-displaced region of the amplicon, the molecular beacon is arranged linearly, with the quencher and fluorophore being spatially separated, emitting fluorescence.

The term "molecular beacon", as used herein, refers to a specific probe comprising a nucleotide sequence, complementary in part to a target nucleotide sequence to be recognised, a fluorophore linked to the 3' or 5' end of the nucleotide sequence, and a quencher linked to the remaining end of the molecule.

The phrase "at least one molecular beacon", as used herein, refers to one or more molecular beacons, depending on the number of viruses and/or specific viral sequences to be detected in the sample. Thus, a different molecular beacon is used in the method of the invention for each different virus and/or specific viral sequence to be detected. In turn, each different molecular beacon comprises a different nucleotide sequence, and a different fluorophore/quencher.

Thus, in some embodiments of the method of the invention, when the virus detection comprises the simultaneous detection of at least 2 viruses and/or when the specific viral sequence detection comprises the simultaneous detection of at least 2 specific viral sequences, the step (c) comprises adding at least 2 molecular beacons. A preferred embodiment of the step (c) of the method of the invention comprises adding 2 molecular beacons. In another preferred embodiment, the step (c) comprises adding 3 molecular beacons.

In the present invention, the nucleotide sequence to be recognised by the molecular beacon is a strand-displaced region of the amplicon. The strand-displaced region recognition is mediated through the hybridization of part of the molecular beacon nucleotide sequence.

The potential opening of the beacon by the sgRNA should be avoided. In this regard, the beacon may not be fully complementary to the displaced strand (only a part of the molecular beacon hybridzes to it). Additionally, the beacon is not fully complementary to the sgRNA, in particular, is not fully complementary to the spacer of the sgRNA.

In a preferred embodiment of the method of the invention, the molecular beacon comprises a nucleotide sequence that is complementary to a 50 to 70% (including the end values of the range) of the strand-displaced region of the amplicon. In a more preferred embodiment of the method of the invention, the molecular beacon comprises a nucleotide sequence that is complementary to a 50% of the strand-displaced region of the amplicon.

In another preferred embodiment of the method of the invention, the molecular beacon comprises a nucleotide sequence that is complementary to a 45 to 70%, preferably, to a 50%of the strand-displaced region of the amplicon, and the sgRNA comprises a sequence which does not hybridize to the molecular beacon.

In another preferred embodiment of the method of the invention, alone or in combination with the rest of the above-mentioned preferred embodiments, the molecular beacon is complementary to a 30% to 50% of the sgRNA spacer.

A preferred embodiment of the method of the invention, comprises an additional step which comprises a cycle of heating and cooling the molecular beacon, prior to be added in the step (c) of the method of the invention.

More preferably, the molecular beacon heating is performed at a temperature of 90 ºC to 100 ºC (including the end values of the range). Even more preferably, the molecular beacon heating is performed at a temperature of 90, 91, 92, 93, 94, 95, 96, 97, 98, 99 or 100 ºC.

In another preferred embodiment, the molecular beacon heating is performed for 1 to 5 min. More preferably, the molecular beacon heating is performed for 1, 2, 3, 4 or 5 min.

In a still more preferred embodiment of the method of the invention, the molecular beacon heating is performed at a temperature of 90 to 100 ºC, preferably 95º C, for 1 to 5 min, preferably 2 min.

In the present invention, "cooling" refers to a refrigeration, preferably slow refrigeration, of the molecular beacon. In a preferred embodiment of the method of the invention, alone or in combination with the rest of the preferred embodiments, the molecular beacon is cooled to 25 ºC.

In a more preferred embodiment, the molecular beacon heating is performed at a temperature of 95ºC for 2 min and cooled to 25 ºC.

The term "hybridization", as it has been previously described, refers to interactions based on base-pairing between two nucleotide sequences. In this step, the molecular beacon, in particular, a part of the molecular beacon, hybridizes to the strand-displaced region of the amplicon.

As it has been described previously, the phrase "strand-displaced region of the amplicon", refers to a region of the amplicon strand which has been displaced by the Cas9 strand-displacement activity.

In a more preferred embodiment of the method of the invention, the nucleotide sequence of the molecular beacon comprises, or consists of, a nucleotide sequence with a sequence identity of, at least, 70, 75, 80, 85, 90, 91, 92, 93, 94, 95, 96, 97, 98 or 99% with sequence SEQ ID NO: 24.
SEQ ID NO: 24
CGCTGATTTTGGGGTCGTAGTTAATCAGCGA

Sequence SEQ ID NO: 24 corresponds to a preferred nucleotide sequence of the molecular beacon able to hybridize with a strand-displaced region of the amplicon obtained from a region of the SARS-CoV-2 N-gene (N1 region). In a still more preferred embodiment of the method of the invention, the nucleotide sequence of the molecular beacon comprises or consists of, sequence SEQ ID NO: 24.

In another more preferred embodiment of the method of the invention, the nucleotide sequence of the molecular beacon comprises, or consists of, a nucleotide sequence with a sequence identity of, at least, 70, 75, 80, 85, 90, 91, 92, 93, 94, 95, 96, 97, 98 or 99% with sequence SEQ ID NO: 25.
SEQ ID NO: 25
CTTCGGAATGTCGCGCGCTTTCTTCCGAAGT

Sequence SEQ ID NO: 25 corresponds to a preferred nucleotide sequence of the molecular beacon able to hybridize with a strand-displaced region of the amplicon obtained from a region of the SARS-CoV-2 N-gene (N2 region). In a still more preferred embodiment of the method of the invention, the nucleotide sequence of the molecular beacon comprises or consists of, sequence SEQ ID NO: 25

In another more preferred embodiment of the method of the invention, the nucleotide sequence of the molecular beacon comprises, or consists of, a nucleotide sequence with a sequence identity of, at least, 70, 75, 80, 85, 90, 91, 92, 93, 94, 95, 96, 97, 98 or 99% with sequence SEQ ID NO: 26.
SEQ ID NO: 26
GCGTACTGCTGCAATATTCGTCCAGTACGC

Sequence SEQ ID NO: 26 corresponds to a preferred nucleotide sequence of the molecular beacon able to hybridize with a strand-displaced region of the amplicon obtained from a region of the SARS-CoV-2 E-gene. In a still more preferred embodiment of the method of the invention, the nucleotide sequence of the molecular beacon comprises or consists of, the sequence SEQ ID NO: 26.

Thus, sequences SEQ ID NO: 24, SEQ ID NO: 25 and SEQ ID NO: 26 correspond to molecular beacon nucleotide sequences able to hybridize with a strand-displaced region of the amplicon obtained from SARS-CoV-2 and/or from a biological sample isolated from a subject infected with SARS-CoV-2.

In another more preferred embodiment of the method of the invention, the nucleotide sequence of the molecular beacon comprises, or consists of, a nucleotide sequence with a sequence identity of, at least, 70, 75, 80, 85, 90, 91, 92, 93, 94, 95, 96, 97, 98 or 99% with sequence SEQ ID NO: 27.
SEQ ID NO: 27
CTCACGTTAACAATATTGCAGCGTCATCATATTGTTAATGTGAGT

Sequence SEQ ID NO: 27 corresponds to a nucleotide sequence of the molecular beacon able to hybridize with a strand-displaced region of the amplicon obtained from SARS-CoV-1 and/or from a biological sample isolated from a subject infected with SARS-CoV-1. In a still more preferred embodiment of the method of the invention, the nucleotide sequence of the molecular beacon comprises or consists of, the sequence SEQ ID NO: 27.

In another more preferred embodiment of the method of the invention, the nucleotide sequence of the molecular beacon comprises, or consists of, a nucleotide sequence with a sequence identity of, at least, 70, 75, 80, 85, 90, 91, 92, 93, 94, 95, 96, 97, 98 or 99% with the sequence SEQ ID NO: 28.
SEQ ID NO: 28
GGCTTTTATGGGTATGTCGCAATATTATTACCTATAAAAGCC

Sequence SEQ ID NO: 28 corresponds to a nucleotide sequence of the molecular beacon able to hybridize with a strand-displaced region of the amplicon obtained from MERS-CoV and/or from a biological sample isolated from a subject infected with MERS-CoV. In a still more preferred embodiment of the method of the invention, the nucleotide sequence of the molecular beacon comprises or consists of, the sequence SEQ ID NO: 28.

In some preferred embodiments of the method of the invention, in particular, in simultaneous detection preferred embodiments, alone or in combination with other preferred embodiments of the method of the invention, the molecular beacons comprise nucleotide sequences with sequence identity of, at least 70, 75, 80, 85, 90, 91, 92, 93, 94, 95, 96, 97, 98 or 99% to nucleotide sequences selected from the list of sequences consisting of: SEQ ID NO:24, SEQ ID NO: 25, SEQ ID NO: 26, SEQ ID NO: 27, SEQ ID NO 28, and any combination thereof.

In some more preferred embodiments of the method of the invention, particularly in simultaneous detection preferred embodiments, alone or in combination with other preferred embodiments of the method of the invention, the molecular beacons nucleotide sequences are selected from the list of sequences consisting of: SEQ ID NO:24, SEQ ID NO: 25, SEQ ID NO: 26, SEQ ID NO: 27, SEQ ID NO 28, and any combination thereof.

As it has been previously explained, the molecular beacon comprises a fluorophore linked to the 3' or 5' end of its nucleotide sequence, and a quencher linked to the remaining end of its nucleotide sequence.

The term "fluorophore", as used herein, refers to a molecule with fluorescence properties. A fluorophore absorbs light energy of a specific wavelength and re-emits light at a longer wavelength. The absorbed wavelengths, energy transfer efficiency, and time before emission depend on both the fluorophore structure and its chemical environment, as the molecule in its excited state interacts with surrounding molecules.

The term "quencher", as used herein, refers to a molecule that absorbs excitation energy from a fluorophore and dissipates the energy as heat. As a person skilled in the art knows, quenchers can be used in molecular biology in conjunction with fluorophores. When both are close together, the fluorophore's emission is suppressed or decreased.

Fluorophores and their cognate quenchers are known in the art and can be selected for this purpose by one having ordinary skill in the art

Examples of quenchers known in the state of the art, that may be used in the present invention are commercial quenchers BHQ-1, BHQ-2, BHQ-3, BBQ-650; ECLIPSE 2,4-dinitrophenyl (DNP), 4-([4'-dimethylamino)phenyl]azo)benzoyl (DABCYL), Iowa Black FQ, and Iowa Black RQ. Thus, in a preferred embodiment of the method of the invention, the quencher is selected from the list consisting of BHQ-1, BHQ-2, BHQ-3, BBQ-650; ECLIPSE 2,4-dinitrophenyl (DNP), 4-([4'-dimethylamino)phenyl]azo)benzoyl (DABCYL), Iowa Black FQ, Iowa Black RQ and any combination thereof. More preferably, the quencher is Iowa Black FQ and/or Iowa Black RQ.

In a still more preferred embodiment, the quencher is Iowa Black FQ when the fluorophores emits at wavelengths below 600 nm. In another still more preferred embodiment, the quencher is Iowa Black RQ when the fluorophores emits at wavelengths above 600 nm.

Examples of fluorophores known in the state of the art, that may be used in the present invention are ATTO-390, ATTO-425, Fluo, FITC, ATTO-495, TET, ATTO-520, Cy3, ROX, Texas Red, ATTO-Rho13, DY480XL, Cy5.5, ATTO-Rho14, IRD700, 2-Aminobenzoyl (Abz), 5-[(2-Aminoethyl)amino], naphthalene-1-sulfonic acid (EDANS), 7-Methoxycoumarin-4-yl)acetyl, FAM, TAMRA, and Cy5.

Thus, in a preferred embodiment of the method of the invention, the fluorophore is selected from the list consisting of: ATTO-390, ATTO-425, Fluo, FITC, ATTO-495, TET, ATTO-520, Cy3, ROX, Texas Red, ATTO-Rho13, DY480XL, Cy5.5, ATTO-Rho14, IRD700, 2-Aminobenzoyl (Abz), 5-[(2-Aminoethyl)amino], naphthalene-1-sulfonic acid (EDANS), 7-Methoxycoumarin-4-yl)acetyl, FAM, TAMRA, and Cy5 and/ any combination thereof. More preferably, the fluorophore is FAM, TAMRA, and/or Cy5.

In a more preferred embodiment of the method of the invention, the fluorophore is Cy5 and the quencher is Iowa Black RQ.

In another more preferred embodiment of the method of the invention, the fluorophore is FAM and the quencher is Iowa Black FQ.

In another more preferred embodiment of the method of the invention, the fluorophore is TAMRA and the quencher is Iowa Black FQ.

As it has been previously described, each different molecular beacon comprises a different a different fluorophore/quencher pair. Thus, in another embodiment of the method of the invention, the fluorophores are selected from the list consisting of FAM, TAMRA, Cy5, and any combination thereof. In another embodiment of the method of the invention, the quenchers are Iowa Black FQ, Iowa Black RQ and/or any combination thereof. In another embodiment of the method of the invention, the fluorophores are FAM, TAMRA and Cy5, and the quenchers are Iowa Black FQ and Iowa Black RQ.

In a preferred embodiment of the method of the invention, the fluorophore is linked, preferably covalently linked, to the 3' end of the nucleotide sequence and the quencher is linked, preferably covalently linked, to the 5' end of the nucleotide sequence.

In another preferred embodiment of the method of the invention, the fluorophore is linked, preferably covalently linked, to the 5' end of the nucleotide sequence and the quencher is linked, preferably covalently linked, to the 3' end of the nucleotide sequence.

As it has been mentioned, the molecular beacon is arranged in a conformation such that:
(i) when it does not hybridize to the strand-displaced region of the amplicon, the molecular beacon is arranged in a hairpin structure, with the quencher and fluorophore located spatially close without emitting fluorescence or emitting a basal fluorescence;
(ii) when it hybridizes to the strand-displaced region of the amplicon, the molecular beacon is arranged linearly, with the quencher and fluorophore being spatially separated, emitting fluorescence.

The adoption of the hairpin structure (also called as "stem loop structure", terms interchangeably used in the present document), is possible due to the specific design of the nucleotide sequence such as a high GC content. As a person skilled in the art knows, the melting temperature of a molecule affects the arrangement of an oligonucleotide molecule. The melting temperature of an oligonucleotide molecule depends on the length of the molecule and its specific nucleotide sequence among other factors.

In a preferred embodiment of the method of the invention, the molecular beacon comprises a nucleotide sequence with a melting temperature of 50ºC or more.

In another preferred embodiment of the method of the invention, the molecular beacon comprises a nucleotide sequence with a melting temperature of, at least, 10 ºC higher than the reaction temperature.

In the present invention, when the molecular beacon does not hybridize to the strand-displaced region of the amplicon, the molecular beacon is arranged in this hairpin structure, with the quencher and the fluorophore spatially close, and quenching phenomenon is produced, producing no level of fluorescence or a basal level of fluorescence.

The term "quenching", as used herein, refers to a physicochemical process that decreases the fluorescence intensity from fluorescent molecules, such as a fluorophore. Quenching may be caused by the interaction between a fluorophore and a quenching molecule or quencher. The complex, once formed, is non-fluorescent or only emit a basal level of fluorescence.

In the present invention, when the molecular beacon hybridizes to the strand-displaced region of the amplicon, the molecular beacon is arranged linearly, with the quencher and fluorophore being spatially separated.

The term "spatially separated", as used herein, refers to a molecular separation between the quencher and the fluorophore that avoids the interaction one each other, caused by the linear arrangement of the molecular beacon, that moves away the quencher from the fluorophore. Thus, in this linear arrangement or conformation of the molecular beacon, the quencher and the fluorophore does not interact, and quenching is not produced, generating a fluorescence emission, or an increase of the fluorescence emission in relation to the fluorescence basal level emission.

In the present invention, the separation between the quencher and the fluorophore is given by the length of the beacon. In a preferred embodiment, the quencher and fluorophore separation is from 0.30 to 0.50 nm per beacon nucleotide, more preferably, 0.34 nm per beacon nucleotide. For instance, if the beacon is 20 nucleotide long, the quencher and fluorophore separation is from 6 to 10 nm, more preferably, 6.8 nm.

Typically, increases of more than 100% in relation to the fluorescence basal level emission can be obtained with suitable beacons and sgRNAs. Basal fluorescence levels, corresponding to the buffer with or without folded beacon, can be subtracted to correct the fluorescence signals, generally resulting in an increase of the gain.

In a preferred embodiment of the method of the invention, the concentration of the molecular beacon added is from 50 to 150 nM (including the ends of the range). Preferably, the concentration of the molecular beacon is from 75 to 120 nM. In a more preferred embodiment of the method of the invention, the concentration of the molecular beacon added in (c) is selected from the list consisting of 75, 80, 85, 90, 95, 100, 105, 110, 115 and 120 nM. Even more preferably, the molecular beacon concentration is 100 nM.

As a person skilled in the art knows, the addition of the molecular beacon may be followed by an incubation period before the following step. In a preferred embodiment of the method of the invention, the incubation period of the ribonucleoprotein-DNA complex with the added molecular beacon is from 1 to 10 min, preferably 5 min.

### Step (d): fluorescence emission detection

A fourth step of the method of the invention, the step (d), comprises measuring, at least, one fluorescent signal and comparing to control values, wherein an increase of the fluorescent signal compared to control values indicates that the target viral nucleotide sequence is present in the sample, hence allowing virus detection and/or specific viral sequence detection.

As a person skilled in the art knows, measuring fluorescent signals can be carried out by techniques known in the art, such as fluorometric techniques or fluorescence techniques. Fluorometric techniques may be based on the use of a fluorometer.

In a preferred embodiment of the method of the invention, the measure of, at least, one fluorescent signal is carried out using a fluorometer.

A "fluorometer" (or "fluorimeter", terms interchangeably used in the present document) is a device used to measure parameters of visible spectrum fluorescence: its intensity and wavelength distribution of emission spectrum after excitation by a certain spectrum of light.

As used herein, "fluorescent signal" refers to a fluorescence emission generated by a fluorophore. As a person skilled in the art knows, a fluorophore absorbs light energy of a certain wavelength and emits that light at a longer wavelength, which may be detected. Moreover, it is also known that, depending on the fluorophore used, excitation and emission wavelength may be different.

Thus, in the invention, when the fluorophore is FAM, excitation may be at 495/12 nm and emission may be at 520/12 nm (green).

When the fluorophore is TAMRA, excitation may be at 557/12 nm and emission may be at 583/12 nm (orange).

When the fluorophore is Cy5, excitation may be at 645/12 nm and emission may be at 670/12 nm (red).

Based on this principle, it is possible for multiplex or simultaneous detection the use of fluorophores with different emission and/or excitation wavelength.

The phrase "at least one fluorescent signal", refers to one or more fluorescent signals. In the method of the invention, each different virus and/or specific viral sequence to be detected in the sample, generates a different fluorescent signal to be measured.

In some embodiments of the method of the invention, when the virus detection comprises the simultaneous detection of at least 2 viruses and/or when the specific viral sequence detection comprises the simultaneous detection of at least 2 specific viral sequences, the step (d) comprises measuring at least 2 fluorescent signals. In a preferred embodiment, the step (d) comprises measuring 2 fluorescent signals. In another preferred embodiment of the method of the invention, the step (d) comprises measuring 3 fluorescent signals.

As it has been previously mentioned, in the step (d) of the method of the invention, fluorescent signal/s measured are compared to control values.

In the present invention, "control values" refers to the fluorescence emission when in the sample is not present any target viral nucleotide sequence and/or is not present the virus or viruses to be detected. In this situation, quenching leads to a basal fluorescence emission or no fluorescence emission occurs.

As it has been also mentioned, in the method of the invention, an increase of the fluorescent signal compared to control values indicates that the target viral nucleotide sequence is present in the sample, hence allowing virus detection and/or specific viral sequence detection.

In the present invention, the phrase "increase of the fluorescent signal compared to control values", refers to a significant increase of the fluorescence emission measured compared to control values. In a preferred embodiment of the method of the invention, the increase of the fluorescent signal is of, at least, 1.5 times compared to control values. In another preferred embodiment of the method of the invention, the increase of the fluorescent signal is of 1.50 times, 1.55 times, 1.60 times, 1.65 times, 1.70 times, 1.75 times, 1.80 times, 1.85 times, 1.90 times, 1.95 times, 2 times, 2.05 times, 2.10 times, 2.15 times, 2.20 times or more times compared to control values.

As it has been described, an increase of the fluorescent signal compared to control values occurs when the nucleotide sequence of the molecular beacon hybridizes to the strand-displaced region of the amplicon. In turn, the amplicon is obtained from the amplification of a target viral nucleotide sequence. Therefore, an increase of the fluorescent signal compared to control values indicates that the target viral nucleotide sequence is present in the sample, hence allowing virus detection and/or specific viral sequence detection.

In a preferred embodiment of the method of the invention, virus detection comprises the detection of, at least, one virus. Preferably, the virus is respiratory virus.

Examples of respiratory viruses include, without limitation to, coronavirus, influenza A virus, influenza B virus, adenovirus, human bocavirus (HBoV), human metapneumovirus (HMPV), parainfluenza virus, respiratory syncytial virus (RSV), rhinovirus and enterovirus.

Thus, in a more preferred embodiment of the method of the invention, at least one respiratory virus is selected from the list consisting of: coronavirus, influenza A virus, influenza B virus, adenovirus, human bocavirus (HBoV), human metapneumovirus (HMPV), parainfluenza virus, respiratory syncytial virus (RSV), rhinovirus, enterovirus, and any combination thereof.

Preferably, the respiratory virus is a coronavirus. Examples of coronavirus include, without limitation to, severe acute respiratory syndrome coronavirus 2 (SARS-CoV-2), severe acute respiratory syndrome coronavirus (SARS-CoV-1), middle east respiratory syndrome coronavirus (MERS-CoV), human coronavirus 229E, human coronavirus NL63, Miniopterus bat coronavirus 1 ,Miniopterus bat coronavirus HKU8, porcine epidemic diarrhea virus, Rhinolophus bat coronavirus HKU2, Scotophilus bat coronavirus 512, bovine coronavirus, human coronavirus OC43, human coronavirus HKU1, murine coronavirus, Pipistrellus bat coronavirus HKU5, Rousettus bat coronavirus HKU9, Tylonycteris bat coronavirus HKU4, hedgehog coronavirus 1, infectious bronchitis virus, beluga whale coronavirus SW1, infectious bronchitis virus, Bulbul coronavirus HKU11, pangolin coronavirus, porcine coronavirus HKU15, WIVI-CoV, SHC014-CoV, bat-SL-CoVZC45, bat-SLCoVZXC21, SARS-CoVGZ02, BtKY72, WIV16, Rs4231, Rs7327, Rs9401, BtRs- BetaCoV/YN2018R, BtRs-BetaCoV/YN2013, Anlong-112, Rf2092, BtRs- BetaCoV/YN2018C, As6526, Rs4247, BtRs-BetaCoV/GX2013, Yunnan2011, BtRI- BetaCoV/SC2018, Shannxi2011, BtRs-BetaCoV/HuB2013, Bat_CoV_279/2005, HKU3-12, HKU3-3, HKU3-7, Longquan-140, RaTG13 and a variant thereof.

Thus, in a still more preferred embodiment of the method of the invention, the coronavirus is selected from the list consisting of: severe acute respiratory syndrome coronavirus 2 (SARS-CoV-2), severe acute respiratory syndrome coronavirus (SARS-CoV-1), middle east respiratory syndrome coronavirus (MERS-CoV), human coronavirus 229E, human coronavirus NL63, Miniopterus bat coronavirus 1 ,Miniopterus bat coronavirus HKU8, porcine epidemic diarrhea virus, Rhinolophus bat coronavirus HKU2, Scotophilus bat coronavirus 512, bovine coronavirus, human coronavirus OC43, human coronavirus HKU1, murine coronavirus, Pipistrellus bat coronavirus HKU5, Rousettus bat coronavirus HKU9, Tylonycteris bat coronavirus HKU4, hedgehog coronavirus 1, infectious bronchitis virus, beluga whale coronavirus SW1, infectious bronchitis virus, Bulbul coronavirus HKU11, pangolin coronavirus, porcine coronavirus HKU15, WIVI-CoV, SHC014-CoV, bat-SL-CoVZC45, bat-SLCoVZXC21, SARS-CoVGZ02, BtKY72, WIV16, Rs4231, Rs7327, Rs9401, BtRs-BetaCoV/YN2018R, BtRs-BetaCoV/YN2013, Anlong-112, Rf2092, BtRs-BetaCoV/YN2018C, As6526, Rs4247, BtRs-BetaCoV/GX2013, Yunnan2011, BtRI-BetaCoV/SC2018, Shannxi2011, BtRs-BetaCoV/HuB2013, Bat_CoV_279/2005, HKU3-12, HKU3-3, HKU3-7, Longquan-140, RaTG13, and any combination thereof..

Even more preferably, the coronavirus is selected from the list consisting of SARS-CoV-2, SARS-CoV-1, MERS-CoV, and any combination thereof.

In another preferred embodiment of the method of the invention, specific viral sequence detection comprises the detection of, at least, one specific viral sequence.

More preferably, the viral sequence is from, at least, one coronavirus, even more preferably wherein the coronavirus is selected from the list consisting of SARS-CoV-2, SARS-CoV-1, MERS-CoV, and any combination thereof.

In another still more preferred embodiment of the method of the invention, the viral specific sequence is selected from the list of sequences consisting of SEQ ID NO: 31, SEQ ID NO: 29, SEQ ID NO: 30, SEQ ID NO: 32, SEQ ID NO: 33, and any combination thereof.

In another still more preferred embodiment of the method of the invention, the viral specific sequence is selected from the list of sequences consisting of SEQ ID NO: 31, SEQ ID NO: 29, SEQ ID NO: 30, and any combination thereof.

The preferred embodiments for "virus", "respiratory virus", "coronavirus" and "viral specific sequence", have been previously described and its definitions and preferred embodiments apply equally hereinafter.

As it has been mentioned, the method of the invention allows the simultaneous/multiplex virus detection in a sample and/or, the simultaneous/multiplex specific viral sequences detection in a sample.

The term "simultaneous detection", as used herein, refers to the detection of more than one virus and/or more than one specific viral sequence in a sample.

Thus, in a preferred embodiment of the method of the invention, virus detection comprises the simultaneous detection of, at least, 2 viruses.

Another preferred embodiment comprises the simultaneous detection of, at least, 2, 3, 4, 5, 6 viruses in a sample. Preferably, virus detection comprises the simultaneous detection of 2 or 3 viruses in a sample.

In a still more preferred embodiment of the method of the invention, virus detection comprises the simultaneous detection of SARS-CoV-2 and SARS-CoV-1.

In another still more preferred embodiment, virus detection comprises the simultaneous detection of SARS-CoV-2 and MERS-CoV.

In another still more preferred embodiment, virus detection comprises the simultaneous detection of SARS-CoV-1 and MERS-CoV.

In another still more preferred embodiment, virus detection comprises the simultaneous detection of SARS-CoV-2, SARS-CoV-1 and MERS-CoV.

In another preferred embodiment of the method of the invention, viral sequence detection comprises the simultaneous detection of, at least, 2 specific viral sequences.

Another preferred embodiment comprises the simultaneous detection of, at least, 2, 3, 4, 5, 6 viral sequences in a sample. Preferably, virus detection comprises the simultaneous detection of 2 or 3 viral sequences in a sample.

In a still more preferred embodiment of the method of the invention, virus detection comprises the simultaneous detection of SEQ ID NO: 31 (E-gene) and SEQ ID NO: 29 (N1-gene region).

In another still more preferred embodiment, virus detection comprises the simultaneous detection of SEQ ID NO: 31 (E-gene) and SEQ ID NO: 30 (N2-gene region).

In another still more preferred embodiment, virus detection comprises the simultaneous detection of SEQ ID NO: 29 (N1-gene region), SEQ ID NO: 30 (N2-gene region).

In another still more preferred embodiment, virus detection comprises the simultaneous detection of SEQ ID NO: 31 (E-gene), SEQ ID NO: 29 (N1-gene region) and SEQ ID NO: 30 (N2-gene region).

### Kit of the invention

Other aspect of the present invention relates to a kit, hereinafter the "kit of the invention", comprising:
- a Cas9 protein,
- at least, one single sgRNA, wherein the sgRNA comprises a nucleotide sequence that hybridizes with a target region of a viral amplicon, and
- at least, one molecular beacon, wherein the molecular beacon comprises a nucleotide sequence that hybridizes with a strand-displaced region of the viral amplicon, a fluorophore linked to the 3' or 5' end of its nucleotide sequence, and a quencher linked to the remaining end of its nucleotide sequence, arranged in a conformation, such that:
   (i) when the molecular beacon does not hybridize with the strand-displaced region of the viral amplicon, the molecular beacon is arranged in a hairpin structure, with the quencher and fluorophore located spatially close without emitting fluorescence or emitting a basal fluorescence;
   (ii) when the molecular beacon hybridizes with the strand-displaced region of the viral amplicon, the molecular beacon is arranged linearly, with the quencher and fluorophore being spatially separated, emitting fluorescence.

In a preferred embodiment of the invention, the kit further comprises the means necessary for carrying out the method of the invention.

As used herein, "means necessary for carrying out the method of the invention" means all those reagents necessary (buffers, enzymes, coenzymes, substrates, etc.), all supports and containers necessary for its implementation and optimisation (plastic tubes, plates, reagents, etc.), as well as other molecules, including nucleic acids, primers, proteins or probes of interest, which enable carrying out the method of the invention or which serve as positive and negative controls.

As in the previous aspect of the invention, the primers may include any suitable primers that amplify target viral nucleotide sequence/s from viral nucleic acid/s. As a person skilled in the art knows, a forward primer and a reverse primer is used for the amplification of a nucleotide sequence to obtain a double-stranded nucleotide sequence, in the present invention, a viral amplicon. Thus, in a more preferred embodiment of the kit of the invention, the means necessary for carrying out the method of the invention comprise, at least, one forward primer, and, at least, one reverse primer.

In an even more preferred embodiment of the kit of the invention, the forward primer, comprises, or consists of, a nucleotide sequence with a sequence identity of, at least 70, 75, 80, 85, 90, 91, 92, 93, 94, 95, 96, 97, 98 or 99% to SEQ ID NO: 1, and the reverse primer which comprises, or consists of, a nucleotide sequence with a sequence identity of , at least 70, 75, 80, 85, 90, 91, 92, 93, 94, 95, 96, 97, 98 or 99% to SEQ ID NO: 2.

In a still more preferred embodiment of the kit of the invention, the forward primer comprises, or consists of, the SEQ ID NO: 1, and the reverse primer comprises, or consists of, the SEQ ID NO: 2.

In an even more preferred embodiment of the kit of the invention the forward primer, comprises, or consists of, a nucleotide sequence with a sequence identity of, at least 70, 75, 80, 85, 90, 91, 92, 93, 94, 95, 96, 97, 98 or 99% to SEQ ID NO: 3, and the reverse primer comprises, or consists of, a nucleotide sequence with a sequence identity of, at least 70, 75, 80, 85, 90, 91, 92, 93, 94, 95, 96, 97, 98 or 99% to SEQ ID NO: 4.

In a still more preferred embodiment of the kit of the invention, the forward primer comprises, or consists of, the SEQ ID NO: 3, and the reverse primer comprises, or consists of, the SEQ ID NO: 4.

In an even more preferred embodiment of the kit of the invention, the forward primer comprises, or consists of, a nucleotide sequence with a sequence identity of, at least 70, 75, 80, 85, 90, 91, 92, 93, 94, 95, 96, 97, 98 or 99% to SEQ ID NO: 5, and the reverse primer comprises, or consists of, a nucleotide sequence with a sequence identity of , at least 70, 75, 80, 85, 90, 91, 92, 93, 94, 95, 96, 97, 98 or 99% to SEQ ID NO: 6.

In a still more preferred embodiment of the kit of the invention, the forward primer comprises, or consists of, the SEQ ID NO: 5, and the reverse primer comprises, or consists of, the SEQ ID NO: 6.

In an even more preferred embodiment of the kit of the invention, the forward primer comprises, or consists of, a nucleotide sequence with a sequence identity of, at least 70, 75, 80, 85, 90, 91, 92, 93, 94, 95, 96, 97, 98 or 99% to SEQ ID NO: 7, and the reverse primer comprises, or consists of, a nucleotide sequence with a sequence identity of , at least 70, 75, 80, 85, 90, 91, 92, 93, 94, 95, 96, 97, 98 or 99% to SEQ ID NO: 8.

In a still more preferred embodiment of the kit of the invention, the forward primer comprises, or consists of, the SEQ ID NO: 7, and the reverse primer comprises, or consists of, the SEQ ID NO: 8.

In an even more preferred embodiment of the kit of the invention, the forward primer, comprises, or consists of, a nucleotide sequence with a sequence identity of, at least 70, 75, 80, 85, 90, 91, 92, 93, 94, 95, 96, 97, 98 or 99% to SEQ ID NO: 9, and the reverse primer comprises, or consists of, a nucleotide sequence with a sequence identity of , at least 70, 75, 80, 85, 90, 91, 92, 93, 94, 95, 96, 97, 98 or 99% to SEQ ID NO: 10.

In a still more preferred embodiment of the kit of the invention, the forward primer comprises, or consists of, the SEQ ID NO: 9, and the reverse primer comprises, or consists of, the SEQ ID NO: 10.

Preferably, the kit of the invention further comprises instructions for carrying out the method of the invention. These instructions may be present in said kit in a variety of forms, one or more of which may be present in the kit. One form in which these instructions may be present is as printed information on a suitable medium or substrate, e.g., a sheet or sheets of paper on which the information is printed, on the kit packaging, on a package insert, and so on. Another medium would be a computer readable medium, e.g. CD, USB, etc., on which the information has been recorded. Another medium that may be present is a website address that can be used via the Internet to access the information at a remote site. Any convenient medium may be present in the kits.

The terms used to define the kit of the invention have already been described in the previous aspect of the present invention and apply equally, as well as its preferred embodiments, to the kit of the invention.

The term "Cas9 protein", has already been described in the previous aspect of the present invention and its definition, as well as all its preferred embodiments, apply equally to the kit of the invention.

Thus, as it has been previously described, in the present invention the Cas9 protein may be any suitable Cas9 protein which comprises strand displacement activity, and which is able to form a complex with a target nucleotide sequence or amplicon, in conjunction with a sgRNA.

In a preferred embodiment of the kit of the invention, the Cas9 protein does not comprise collateral catalytic activity. Preferably, the Cas9 protein is from *Streptococcus pyogenes*

In a preferred embodiment of the kit of the invention, the *Streptococcus pyogenes* Cas9 is selected from the list consisting of: wild type Cas9 nuclease, Cas9 H840A nickase and catalytically dead Cas9.

"Wild type Cas9 nuclease", is a Cas9 protein from *Streptococcus pyogenes.* In a more preferred embodiment of the kit of the invention, the wild type Cas9 nuclease comprises, or consists of, an amino acid sequence with a sequence identity of, at least, 70, 75, 80, 85, 90, 91, 92, 93, 94, 95, 96, 97, 98 or 99% to sequence SEQ ID NO: 16.

Sequence SEQ ID NO: 16, as it has been previously mentioned, corresponds to the *Streptococcus pyogenes* wild type Cas9 amino acid sequence. In an even more preferred embodiment of the kit of the invention, wild type Cas9 nuclease comprises, or consists of, amino acid sequence SEQ ID NO: 16.

"Cas9 H840A nickase" (or Cas9n) refers a partially catalytically inactive form of Cas9 working like a nickase that has the H840A mutation (in the HNH domain), and which only cleaves the non-targeted strand of the amplicon nucleotide sequence. In another more preferred embodiment of the kit of the invention, the Cas9 H840A nickase comprises, or consists of, an amino acid sequence with a sequence identity of, at least, 70, 75, 80, 85, 90, 91, 92, 93, 94, 95, 96, 97, 98 or 99% with sequence SEQ ID NO: 17.

Sequence SEQ ID NO: 17, corresponds to the *Streptococcus pyogenes* Cas9 H840A nickase amino acid sequence. In an even more preferred embodiment of the kit of the invention, Cas9 H840A nickase comprises, or consists of, amino acid sequence SEQ ID NO: 17.

"Catalytically dead Cas9" (or dCas9) refers to a catalytically dead Cas9 protein that lacks endonuclease activity, and which does not produce any cleavage. In another more preferred embodiment of the kit of the invention, the catalytically dead Cas9 comprises, or consists of, an amino acid sequence with a sequence identity of, at least, 70, 75, 80, 85, 90, 91, 92, 93, 94, 95, 96, 97, 98 or 99% with sequence SEQ ID NO: 18.

Sequence SEQ ID NO: 18, corresponds to the *Streptococcus pyogenes* catalytically dead Cas9 amino acid sequence. In an even more preferred embodiment of the kit of the invention, catalytically dead Cas9 comprises, or consists of, the amino acid sequence SEQ ID NO: 18.

The term "sgRNA", has already been described in the previous aspect of the present invention and its definition, as well as all its preferred embodiments, apply equally to the kit of the invention.

Thus, in another preferred embodiment of the kit of the invention, alone or in combination with the rest of the preferred embodiments, the sgRNA comprises a nucleotide sequence that hybridizes with a target region of an amplicon obtained from a respiratory virus and/or from a biological sample isolated from a subject infected with a respiratory virus.

In another preferred embodiment of the kit of the invention, alone or in combination with the rest of the preferred embodiments, the sgRNA comprises a nucleotide sequence that hybridizes with an amplicon obtained from a coronavirus and/or from a biological sample isolated from a subject infected with a coronavirus.

In another preferred embodiment of the kit of the invention, alone or in combination with the rest of the preferred embodiments, the sgRNA comprises a nucleotide sequence that hybridizes with target region of the amplicon obtained from SARS-CoV-2 and/or from a biological sample isolated from a subject infected with SARS-CoV-2.

In another preferred embodiment of the kit of the invention, alone or in combination with the rest of the preferred embodiments, the sgRNA comprises a nucleotide sequence that hybridizes with target region of the amplicon obtained from SARS-CoV-1 and/or from a biological sample isolated from a subject infected with SARS-CoV-1.

In another preferred embodiment of the kit of the invention, alone or in combination with the rest of the preferred embodiments, the sgRNA comprises a nucleotide sequence that hybridizes with target region of the amplicon obtained from MERS-CoV and/or from a biological sample isolated from a subject infected with MERS-CoV.

In another preferred embodiment, the kit of the invention, comprises 2 sgRNAs wherein the sgRNAs are sgRNAs which comprises nucleotide sequences that hybridize with target region of amplicons obtained from SARS-CoV-2, SARS-CoV-1, MERS-CoV or any combination thereof.

In another preferred embodiment, the kit of the invention, comprises 3 sgRNAs wherein the sgRNAs are sgRNAs which comprises nucleotide sequences that hybridize with target region of amplicons obtained from SARS-CoV-2, SARS-CoV-1, MERS-CoV or any combination thereof.

In another preferred embodiment of the kit of the invention, alone or in combination with the rest of the preferred embodiments, the sgRNA comprises a nucleotide sequence that hybridizes with a target region of the SARS-CoV-2 E-gene amplicon.

In another preferred embodiment of the kit of the invention, alone or in combination with the rest of the preferred embodiments, the sgRNA comprises a nucleotide sequence that hybridizes with a target region of the SARS-CoV-2 N-gene amplicon. More preferably, the sgRNA comprises a nucleotide sequence that hybridizes with the N1 region or N2 region of the SARS-CoV-2 N-gene amplicon.

In another preferred embodiment, the kit of the invention, comprises 2 sgRNAs wherein the sgRNAs are sgRNAs which comprise nucleotide sequences that hybridize with target region of the SARS-CoV-2 E-gene amplicon, N1 region of the SARS-CoV-2 N-gene amplicon, N2 region of the SARS-CoV-2 N-gene amplicon, or any combination thereof.

In another preferred embodiment, the kit of the invention, comprises 3 sgRNAs wherein the sgRNAs are sgRNAs which comprise nucleotide sequences that hybridize with target region of the SARS-CoV-2 E-gene amplicon, N1 region of the SARS-CoV-2 N-gene amplicon, N2 region of the SARS-CoV-2 N-gene amplicon, or any combination thereof.

In a more preferred embodiment of the kit of the invention, the sgRNA comprises, or consists of, a nucleotide sequence with a sequence identity of, at least, 70, 75, 80, 85, 90, 91, 92, 93, 94, 95, 96, 97, 98 or 99% to sequence SEQ ID NO: 19, SEQ ID NO: 20, SEQ ID NO: 21, SEQ ID NO: 22, or SEQ ID NO: 23.

In an even more preferred embodiment of the kit of the invention, the sgRNA comprises, or consists of, the sequence SEQ ID NO: 19, SEQ ID NO: 20, SEQ ID NO: 21, SEQ ID NO: 22 or SEQ ID NO: 23.

In another preferred embodiment, the kit of the invention, comprises 2 sgRNAs wherein the sgRNAs are sgRNAs which comprise or consist of nucleotide sequences with a sequence identity of, at least, 70, 75, 80, 85, 90, 91, 92, 93, 94, 95, 96, 97, 98 or 99% to sequence SEQ ID NO: 19, SEQ ID NO: 20, SEQ ID NO: 21, SEQ ID NO : 22, SEQ ID NO : 23, or any combination thereof.

In another more preferred embodiment, the kit of the invention, comprises 2 sgRNAs wherein the sgRNAs comprise, or consist of, sequences SEQ ID NO: 19, SEQ ID NO: 20, SEQ ID NO: 21, SEQ ID NO: 22, SEQ ID NO : 23, or any combination thereof.

In another preferred embodiment, the kit of the invention comprises 3 sgRNAs wherein the sgRNAs are sgRNAs which comprise or consist of nucleotide sequences with a sequence identity of, at least, 70, 75, 80, 85, 90, 91, 92, 93, 94, 95, 96, 97, 98 or 99% to sequence SEQ ID NO: 19, SEQ ID NO: 20, SEQ ID NO: 21, SEQ ID NO : 22, SEQ ID NO : 23, or any combination thereof.

In another more preferred embodiment, the kit of the invention, comprises 3 sgRNAs wherein the sgRNAs comprise, or consist of, sequences SEQ ID NO: 19, SEQ ID NO: 20, SEQ ID NO: 21, SEQ ID NO: 22, SEQ ID NO: 23, or any combination thereof.

The term "molecular beacon" has already been described in the previous aspect of the present invention and its definition, as well as all its preferred embodiments, apply equally to the kit of the invention.

Thus, another preferred embodiment of the kit of the invention, alone or in combination with the rest of the preferred embodiments, the nucleotide sequence of the molecular beacon comprises, or consists of, a nucleotide sequence with a sequence identity of, at least, 70, 75, 80, 85, 90, 91, 92, 93, 94, 95, 96, 97, 98 or 99% with sequence SEQ ID NO: 24, SEQ ID NO: 25, SEQ ID NO: 26, SEQ ID NO: 27, or SEQ ID NO: 28.

In an even more preferred embodiment of the kit of the invention, the nucleotide sequence of the molecular beacon comprises, or consists of, comprises or consists of, the sequence SEQ ID NO: 24, SEQ ID NO: 25, SEQ ID NO: 26, SEQ ID NO: 27 or SEQ ID NO: 28.

In another preferred embodiment, the kit of the invention, comprises 2 molecular beacons wherein the nucleotide sequences of the molecular beacon are sequences which comprise or consist of nucleotide sequences with a sequence identity of, at least, 70, 75, 80, 85, 90, 91, 92, 93, 94, 95, 96, 97, 98 or 99% to sequence SEQ ID NO: 24, SEQ ID NO: 25, SEQ ID NO: 26, SEQ ID NO: 27, SEQ ID NO: 28, or any combination thereof.

In another more preferred embodiment, the kit of the invention, comprises 2 molecular beacons wherein the nucleotide sequences of molecular beacons comprise, or consist of, sequences SEQ ID NO: 24, SEQ ID NO: 25, SEQ ID NO: 26, SEQ ID NO: 27, SEQ ID NO: 28, or any combination thereof.

In another preferred embodiment, the kit of the invention comprises 3 molecular beacons wherein the nucleotide sequences of molecular beacons comprise or consist of nucleotide sequences with a sequence identity of, at least, 70, 75, 80, 85, 90, 91, 92, 93, 94, 95, 96, 97, 98 or 99% to sequence SEQ ID NO: 24, SEQ ID NO: 25, SEQ ID NO: 26, SEQ ID NO: 27, SEQ ID NO: 28, or any combination thereof.

In another more preferred embodiment, the kit of the invention, comprises 3 molecular beacons wherein the nucleotide sequences of molecular beacons comprise or consist of, sequences SEQ ID NO: 24, SEQ ID NO: 25, SEQ ID NO: 26, SEQ ID NO: 27, SEQ ID NO: 28, or any combination thereof.

The terms used to define the kit of the invention have been previously described in the method of the invention, and apply equally, as well as their preferred embodiments, to the present aspect.

### DESCRIPTION OF THE DRAWINGS

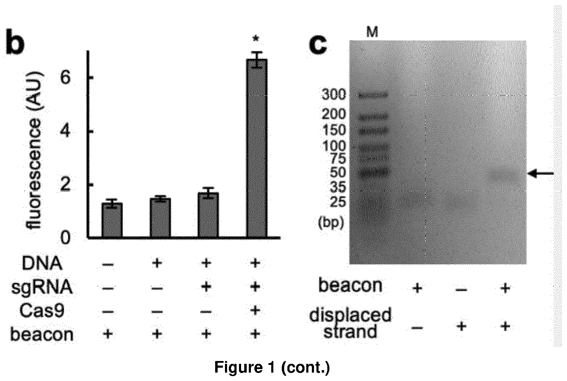

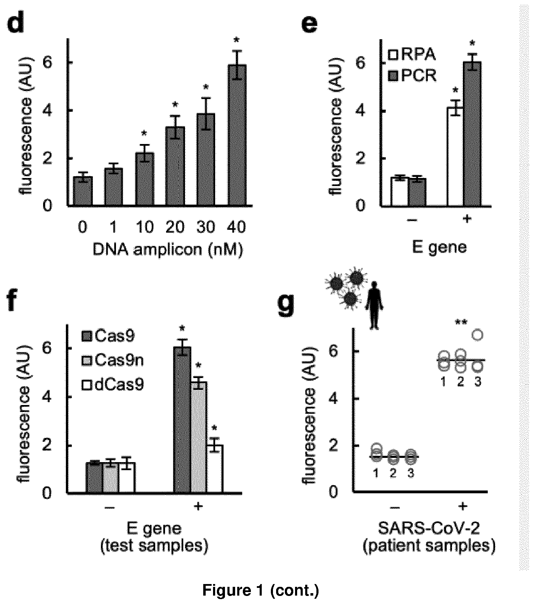

**Figure 1****. SARS-CoV-2 detection through a CRISPR-Cas9-based strand displacement reaction.** a) Schematics of the global reaction of amplification and detection of a DNA product from SARS-CoV-2 E gene (PCR primers drawn at the ends), containing a PAM for Cas9 recognition. A preassembled CRISPR-Cas9 ribonucleoprotein targeting the amplicon was then able to displace a strand so that the molecular beacon could interact with and change its conformation. The molecular beacon was labelled with the fluorophore Cy5 (sun icon) in the 3' end and the dark quencher IB_{RQ} (moon icon) in the 5' end. Wobble base pairs denoted by dots. b) Fluorescence-based characterization of the detection. c) Gel electrophoretic assay to reveal the interaction between the molecular beacon and the displaced strand from the DNA amplicon (the arrow marks the intermolecular complex). M: molecular marker; bp: base pair. d) Effect of the DNA amplicon concentration on the output fluorescence signal. e) Detection of the DNA amplicon generated by PCR or RPA (isothermal method). f) Effect of different versions of Cas9 (Cas9, Cas9n, or dCas9) on the output signal. g) Detection of SARS-CoV-2 in patient samples (3 patients, 3 reactions per patient). Unless specified, amplifications performed by PCR. Error bars correspond to standard deviations (*n* = 3). *Statistical significance with test samples (Welch's *t*-test, two-tailed *P* < 0.05). **Statistical significance with patient samples (Welch's *t*-test, two-tailed *P*< 0.001). Sequences: amplicon (SEQ ID NO: 13), sgRNA (SEQ ID NO: 21), molecular beacon nucleotide sequence (SEQ ID NO: 26), primers (SEQ ID NO: 5 and SEQ ID NO: 6), Cas 9 (wild type nuclease; SEQ ID NO: 16), Cas9n (SEQ ID NO: 17); dCas9 (SEQ ID NO: 18).

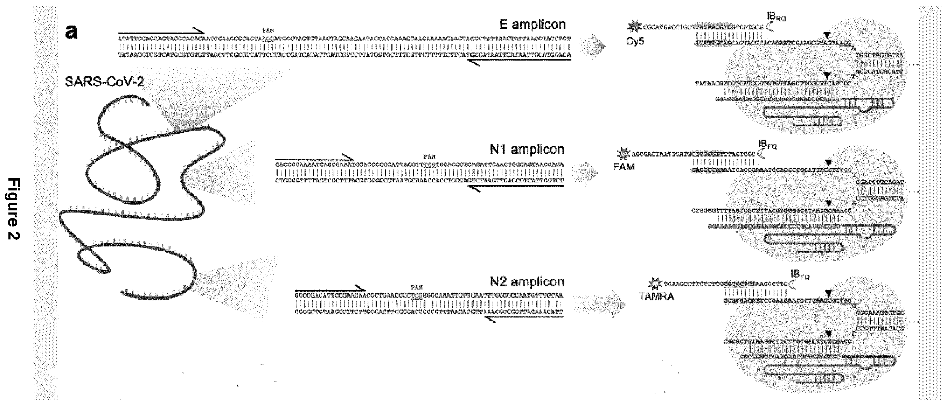

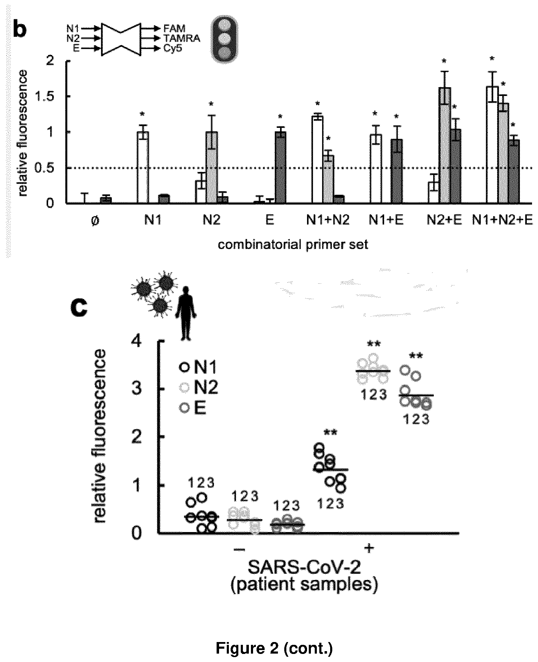

**Figure 2****. Multiplexed SARS-CoV-2 detection through CRISPR-Cas9-based strand displacement reactions.** a) Schematics of the global reactions of amplification and detection of three different DNA products from SARS-CoV-2 (from E and N genes, PCR primers drawn at the ends), containing each a PAM for Cas9 recognition. Preassembled CRISPR-Cas9 ribonucleoproteins targeting the amplicons and appropriate molecular beacons were used for the detection. The molecular beacons were labelled with Cy5 and IB_{RQ} (for E detection), FAM and IB_{FQ} (for N1 detection), and TAMRA and IB_{FQ} (for N2 detection). Wobble base pairs denoted by dots. IB_{RQ}: Iowa Black RQ; IB_{FQ:} Iowa Black FQ. b) Fluorescence-based characterization of the detection by performing amplifications with combinatorial sets of primers. c) Multiplexed detection of SARS-CoV-2 in patient samples (3 patients, 3 reactions per patient). Amplifications performed by PCR. The fluorescence values of the closed-form beacons were subtracted to correct the signals, which were then normalized by the values of the positive singleplex cases. Error bars correspond to standard deviations (*n* = 3). *Statistical significance with test samples (Welch's *t*-test, two-tailed *P* < 0.05, and relative fluorescence > 0.5). **Statistical significance with patient samples (Welch's *t*-test, two-tailed *P* < 0.001). Sequences provided in "Dataset sequence" section. Concretely in this figure: E-amplicon sequence: SEQ ID NO: 13; N1-amplicon sequence: SEQ ID NO: 11; N2-amplicon sequence: SEQ ID NO: 12; N1 primers: SEQ ID NO: 1 and SEQ ID NO: 2; N2 primers: SEQ ID NO: 3 and SEQ ID NO: 4; E primers: SEQ ID NO: 5 and SEQ ID NO: 6; Molecular beacon sequence hybridizing to E-amplicon: SEQ ID NO: 26; Molecular beacon sequence hybridizing to N1-amplicon: SEQ ID NO: 24; Molecular beacon sequence hybridizing to N2-amplicon: SEQ ID NO: 25.

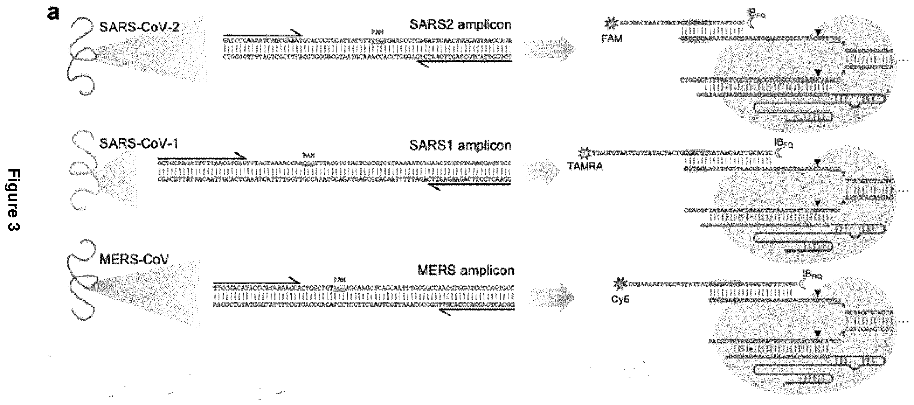

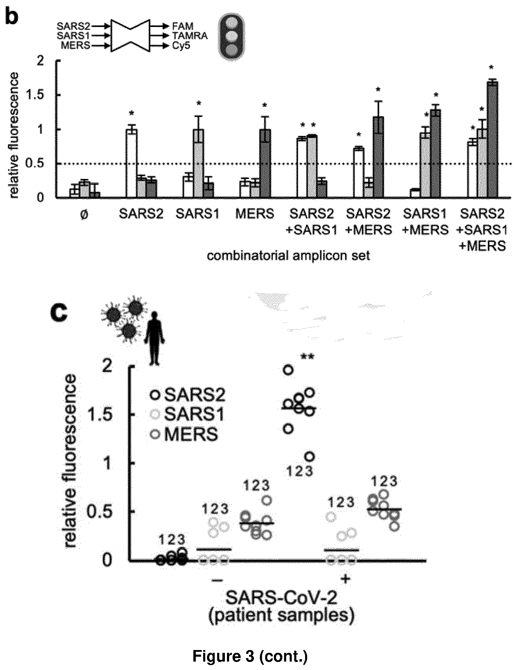

**Figure 3****. Multiplexed coronavirus detection through CRISPR-Cas9-based strand displacement reactions.** a) Schematics of the global reactions of amplification and detection of DNA products from SARS-CoV-2, SARS-CoV-1, and MERS-CoV (PCR primers drawn at the ends), containing each a PAM for Cas9 recognition. Preassembled CRISPR-Cas9 ribonucleoproteins targeting the amplicons and appropriate molecular beacons were used for the detection. The molecular beacons were labelled with FAM and IB_{FQ} (for SARS-CoV-2 detection), TAMRA and IB_{FQ} (for SARS-CoV-1 detection), and Cy5 and IB_{RQ} (for MERS-CoV detection). Wobble base pairs denoted by dots. b) Fluorescence-based characterization of the detection by performing amplifications with combinatorial sets of DNA amplicons. c) Differential detection of SARS-CoV-2 in patient samples (3 patients, 3 reactions per patient). Amplifications performed by PCR. The fluorescence values of the closed-form beacons were subtracted to correct the signals, which were then normalized by the values of the positive singleplex cases. Error bars correspond to standard deviations (*n* = 3). *Statistical significance with test samples (Welch's *t*-test, two-tailed *P* < 0.05, and relative fluorescence > 0.5). **Statistical significance with patient samples (Welch's *t*-test, two-tailed *P* < 0.001). Sequences provided in "Dataset sequence" section. Concretely in this figure: SARS2 amplicon (SEQ ID NO: 11); SARS1 amplicon (SEQ ID NO: 14); MERS amplicon (SEQ ID NO: 15); Molecular beacon sequence hybridizing to SARS2 amplicon (SEQ ID NO: 24); Molecular beacon sequence hybridizing to SARS1 amplicon (SEQ ID NO: 27); Molecular beacon sequence hybridizing to MERS amplicon (SEQ ID NO: 28).

### Examples

### 1. METHODS

**Test samples.** For single detection, a test sample was generated with a plasmid containing the SARS-CoV-2 E gene at 10³ copies/µL. Additional test samples were generated for multiplexed detection. First, a test sample was generated by mixing two plasmids containing the SARS-CoV-2 N and E genes each at 10³ copies/µL. Second, different combinatorial samples of the three DNA amplicons from different viruses were prepared. The DNA amplicon from SARS-CoV-2 was generated by PCR from the aforementioned plasmid with the CDC N1 primers, and the DNA amplicons from SARS-CoV-1 and MERS-CoV were chemically synthesized. Sequences of the amplicons provided below in "Dataset sequence" section, specifically in Table 2.

**Patient samples.** Nasopharyngeal swabs corresponding to infected and non-infected patients with SARS-CoV-2 (RT-qPCR diagnostics) were obtained from the Clinic University Hospital of Valencia (Spain). Samples were inactivated by heat shock (30 min at 60 ºC) before proceeding. No RNA extraction was performed.

**Primers.** The Centers for Disease Control and Prevention (CDC) N1 and N2 primers were used to amplify two different N gene regions from SARS-CoV-2, and the Charité E-Sarbeco primers were used to amplify one E gene region [Vogels CB et al. (2020) Analytical sensitivity and efficiency comparisons of SARS-CoV-2 RT-qPCR primer-probe sets. Nat Microbiol 5, 1299-1305.]. In addition, a genomic region from SARS-CoV-1 was amplified with newly designed primers, and a region from MERS-CoV could be amplified with the previously designed MERS-related N2 primers (Lu X, Whitaker B, Sakthivel SKK, Kamili S, Rose LE, et al. (2014) Real-time reverse transcription-PCR assay panel for Middle East respiratory syndrome coronavirus. J Clin Microbiol 52, 67-75.). Sequences provided below in "Dataset sequence" section, specifically in Table 1 (primers) and Table 6 (viral sequences).

**CRISPR elements.** Three versions of *S*. *pyogenes* Cas9 were used: the wild-type nuclease (Cas9), the Cas9 H840A nickase (Cas9n), and the catalytically dead Cas9 protein (dCas9). In addition, sgRNAs were generated by *in vitro* transcription with the TranscriptAid T7 high yield transcription kit (Thermo) from DNA templates. sgRNAs were then purified by using the RNA clean and concentrator column (Zymo) and quantified in a NanoDrop. Sequences provided below in "Dataset sequence" section, specifically in Table 3 (Cas9 proteins) and Table 4 (sgRNAs).

**Molecular beacons.** Different DNA oligonucleotides folding into a stem-loop structure and appropriately labelled were designed to hybridize with the displaced DNA strands from the CRISPR reactions. These probes were designed to have a seed region in the loop and of high GC content, as well as a melting temperature higher than 50 ºC. The correct folding and hybridization ability (with the target DNA, but not with the sgRNA) were checked with NUPACK (Zadeh JN et al. (2011) NUPACK: analysis and design of nucleic acid systems. J Comput Chem 32, 170-173). Molecular beacons were labelled in their 5' end with a dark quencher (Iowa Black FQ or Iowa Black RQ) and in their 3' end with a fluorophore (FAM, TAMRA, or Cy5). To ensure appropriate folding, molecular beacons were heated at 95 ºC for 2 min and then cooled slowly to 25 ºC prior to their use in the CRISPR reactions. Sequences provided below in "Dataset sequence" section, specifically in Table 5.

**Nucleic acid amplification by PCR.** With test samples, 250 nM of forward and reverse primers, 200 µM dNTPs (NZYTech), 0.02 U/µL Phusion high-fidelity DNA polymerase (Thermo), and 1x Phusion buffer were mixed for a total volume of 20 µL (adjusted with RNase-free water). With patient samples, 500 U RevertAid (Thermo) and 50 U RNase inhibitor (Thermo) were added to the mix. Reactions were incubated in a thermocycler (Eppendorf). The protocol was 98 ºC for 30 s for denaturation, 35 cycles of 98 ºC for 10 s, 62 ºC for 10 s, and 72 ºC for 5 s for amplification, and finally 72 ºC for 2 min for extension. PCR products were purified by using a DNA clean and concentrator column (Zymo).

**Nucleic acid amplification by RPA.** The TwistAmp basic kit (TwistDX) was used. With test samples, 250 nM of forward and reverse primers was added to 29.5 µL of rehydration buffer for a total volume of 43.4 µL (adjusted with RNase-free water). With patient samples, 500 U RevertAid (Thermo) and 50 U RNase inhibitor (Thermo) were added to the mix. The TwistAmp basic reaction pellet was resuspended with this volume, and 21.7 µL in addition to 2 µL of sample were used per reaction. To start the reaction, 280 mM magnesium acetate was added. Reactions were incubated at 40 ºC for 5 min, then vortexed and spun, and reincubated for additional 25 min. RPA products were purified by using the DNA clean and concentrator column.

**CRISPR-Cas9-based detection.** CRISPR reactions were performed in 1x TAE buffer pH 8.5 (Invitrogen), 0.05% Tween 20 (Merck), and 12.5 mM MgCl₂ (Merck) at a final volume of 20 µL. The CRISPR-Cas9 ribonucleoprotein, previously assembled at room temperature for 30 min, was added at 100 nM. 40 nM of amplified DNA (otherwise specified) was used per reaction. Reactions were incubated at 37 ºC for 20 min in a thermocycler (Eppendorf). The molecular beacon was added afterwards at 100 nM, followed by 5 min incubation.

**Fluorometry.** Reaction volumes were loaded in a black 384-well microplate with clear bottom (Corning), which was then placed in a fluorometer (Varioskan Lux, Thermo) to measure green, orange, and red fluorescence (measurement time of 100 ms, automatic range, and top optics). For FAM, excitation was at 495/12 nm and emission at 520/12 nm (green); for TAMRA, excitation was at 557/12 nm and emission at 583/12 nm (orange); and for Cy5, excitation was at 645/12 nm and emission at 670/12 nm (red).

**Gel electrophoresis.** Nucleic acid species were introduced at 7.5 µM each in 20 µL of the CRISPR reaction buffer and were incubated for 30 min at room temperature. Samples were loaded on a 3% agarose gel prepared with 0.5x TBE buffer, which was run for 45 min at room temperature (110 V). The gel was stained using RealSafe (Durviz). The GeneRuler ultra-low range DNA ladder (10-300 bp, Thermo) was used as a marker.

### 2. RESULTS

### 2.1. SARS-CoV-2 detection through CRISPR-Cas9 based strand displacement

A suitable DNA amplicon (SEQ ID NO: 13) was generated by PCR from a test sample based on the SARS-CoV-2 E gene. The amplified material was purified in a column to remove the primers in order to avoid potential interferences with the molecular beacon.

An sgRNA (SEQ ID NO: 21) was designed to exploit a PAM located at an appropriate position (Figure 1a), *in vitro* transcribed from a DNA template, and assembled with a Cas9 (SEQ ID NO: 16) given from a commercial preparation. In turn, a molecular beacon appropriately designed was chemically synthesized, labelling its 3' end with the fluorophore Cy5 and its 5' end with the dark quencher Iowa Black RQ (see Table 5, nucleotide sequence SEQ ID NO: 26) of the molecular beacon used. Then, the sgRNA-Cas9 ribonucleoprotein was added to the reaction to target the amplified DNA and the beacon to produce a red fluorescent signal upon interaction with the displaced strand (i.e., upon detection of the nucleic acid of interest). Remarkably, this approach displayed good performance, with a dynamic range of more than 3-fold change in absolute red fluorescence and no apparent opening of the beacon in response to the DNA amplicon or the sgRNA alone (Figure 1b). The ability of the beacon to interact with the displaced strand was also assessed by agarose gel electrophoresis (Figure 1c).

Furthermore, it was performed a set of reactions with increasing concentrations of DNA amplicon, observing proportionality between the input and output signals (Figure 1d). Thus, the present approach might be used to quantify a given DNA in the sample ranging from the nanomolar scale.

Next, it was tested the ability of using RPA instead of PCR to generate the DNA amplicon, as this is important to achieve point-of-care applications. The results indicate that both methods are suitable (Figure 1e), having used the very same primers (SEQ ID NO: 5 and SEQ ID NO: 6). A slightly higher fluorescent signal in presence of the target DNA was produced with PCR, but the pre-amplification process was faster with RPA.

In addition, it was inspected the impact of the catalytic activity of Cas9 on the performance of the approach. To this end, three different versions of Cas9 were used: the wild-type nuclease (SEQ ID NO: 16), the Cas9 H840A nickase (Cas9n; SEQ ID NO: 17), which only cleaves the non-targeted strand, and the catalytically dead Cas9 protein (dCas9; SEQ ID NO: 18), which does not produce any cleavage. Both, wild-type Cas9 and Cas9n, produced a substantial fold change in fluorescence upon detection, although higher in the case of wild-type Cas9 (Figure 1f). However, dCas9 failed in reaching such a performance, despite a significant differential readout was still possible. Arguably, the cleavage of the non-targeted strand confers more translational and rotational freedom to facilitate the interaction with the beacon.

Motivated by these results, the approach was applied to detect SARS-CoV-2 in patient samples. Nasopharyngeal swabs from people diagnosed as positive or negative in viral infection by RT-qPCR in the hospital were collected]. After RT-PCR amplification (without RNA extraction), the approach allowed to obtain marked differential readouts that were useful to discriminate the presence of the virus (Figure 1g). These results demonstrate the ability of the approach to perform clinical diagnostics in a simple and effective way.

### 2.2. Simultaneous detection of different genomic SARS-CoV-2 region through CRISPR-Cas9 based strand displacement

Then, it was performed the simultaneous detection of different genomic regions of SARS-CoV-2. This is important to minimize the rate of false positives. The Centers for Disease Control and Prevention (CDC) N1 and N2 primers (SEQ ID NO: 1, SEQ ID NO: 2; SEQ ID NO: 3, and SEQ ID NO: 4) were used together with the Charité E-Sarbeco primers (SEQ ID NO: 5 and SEQ ID NO: 6) to generate three different DNA amplicons (SEQ ID NO: 11, SEQ ID NO: 12 and SEQ ID NO: 13) by PCR from a test sample based on the SARS-CoV-2 N and E genes. Suitable PAMs were found in these amplicons (Figure 2a).

The corresponding sgRNAs and molecular beacons were designed according to the aforementioned specifications (see more information in Table 4 and 5 respectively). The new beacons to detect the N1 and N2 amplicons were labelled with the fluorophores FAM and TAMRA, respectively, in their 3' end and with the dark quencher Iowa Black FQ in their 5' end. These fluorophores are compatible with Cy5 (used to detect the E-Sarbeco amplicon). Notably, the present approach allowed the precise detection of the different genomic regions having performed a series of combinatorial amplifications, with a minimal 3-fold change in relative fluorescence (Figure 2b).

In addition, a multiplexed RT-PCR amplification was performed over patient samples with all primers. A positive signal in the three fluorescence channels was given in the case of patients diagnosed as infected by SARS-CoV-2 in the hospital (Figure 2c).

### 2.3. Multiplex virus detection through CRISPR-Cas9 based strand displacement

Finally, it was assessed the ability of the present approach to detect simultaneously different viruses in the sample. Such a multiplexed detection is important because it can allow performing differential diagnostics and uncovering mixed infections. The inventors focused on three different coronaviruses: SARS-CoV-2, SARS-CoV-1, and Middle East respiratory syndrome coronavirus also called as MERS-CoV (viral amplicon sequences shown in Table 2).

The CDC N1 primers were considered for SARS-CoV-2, new primers were designed for SARS-CoV-1, and previously designed primers were taken for MERS-CoV [Lu X, et al., Whitaker B. et al., (2014) Real-time reverse transcription-PCR assay panel for Middle East respiratory syndrome coronavirus. J Clin Microbiol 52, 67-75.]. It was checked that each pair of primers only aligns with the cognate genome, so they are useful to perform multiplexed amplifications (primer sequences shown in Table 1). Suitable PAMs were found in the corresponding amplicons (Figure 3a).

We then designed new sgRNAs (sgRNAs sequences shown in Table 4) and beacons (beacons nucleotide sequences shown in Table 5) to detect SARS-CoV-1 (signal from fluorophore TAMRA) and MERS-CoV (signal from fluorophore Cy5). Notably, the approach allowed the precise detection of the different DNA amplicons added in a combinatorial way, with a minimal 3-fold change in relative fluorescence as before (Figure 3b). In addition, it was performed a multiplexed RT-PCR amplification over patient samples with all primers. A positive signal was given only in the green fluorescence channel, corresponding to SARS-CoV-2, in the case of patients diagnosed as infected in the hospital (Figure 3c). Collectively, these results indicate that the present approach is a suitable method to achieve a direct multiplexed detection of nucleic acids with no need of gel electrophoresis.

### 3. DATASET SEQUENCES

**Table 1. Primers used.**

| **Virus** | **Gene** | **Primer** | **Sequence 5' - 3'** |
|---|---|---|---|
| SARS-CoV-2 | Nucleocapsid | N1 Forward | GACCCCAAAATCAGCGAAAT (SEQ ID NO: 1) |
| SARS-CoV-2 | Nucleocapsid | N1 Reverse | TCTGGTTACTGCCAGTTGAATCTG (SEQ ID NO: 2) |
| SARS-CoV-2 | Nucleocapsid | N2 Forward | TTACAAACATTGGCCGCAAA (SEQ ID NO: 3) |
| SARS-CoV-2 | Nucleocapsid | N2 Reverse | GCGCGACATTCCGAAGAA (SEQ ID NO: 4) |
| SARS-CoV-2 | Envelope protein | E Forward | ACAGGTACGTTAATAGTTAATAGCGT (SEQ ID NO: 5) |
| SARS-CoV-2 | Envelope protein | E Reverse | ATATTGCAGCAGTACGCACACA (SEQ ID NO: 6) |
| SARS-CoV-1 | Envelope protein | E Forward | GCTGCAATATTGTTAACGTGAG (SEQ ID NO: 7) |
| SARS-CoV-1 | Envelope protein | E Reverse | GGAACTCCTTCAGAAGAGTTC (SEQ ID NO: 8) |
| MERS-CoV | Nucleocapsid | N2 Forward | GGCACTGAGGACCCACGTT (SEQ ID NO: 9) |
| MERS-CoV | Nucleocapsid | N2 Reverse | TTGCGACATACCCATAAAAGCA (SEQ ID NO: 10) |

**Table 2. Amplicons sequences.**

| **Virus** | **Gene** | **Region** | **Sequence 5' - 3'** |
|---|---|---|---|
| SARS-CoV-2 | Nucleocapsid | N1 | GACCCCAAAATCAGCGAAATGCACCCCGCATTAC GTTTGGTGGACCCTCAGATTCAACTGGCAGTAAC CAGA (SEQ ID NO: 11) |
| SARS-CoV-2 | Nucleocapsid | N2 | TTACAAACATTGGCCGCAAATTGCACAATTTGCCC CCAGCGCTTCAGCGTTCTTCGGAATGTCGCGC (SEQ ID NO: 12) |
| SARS-CoV-2 | Envelope protei n | E | ACAGGTACGTTAATAGTTAATAGCGTACTTCTTTT TCTTGCTTTCGTGGTATTCTTGCTAGTTACACTAG CCATCCTTACTGCGCTTCGATTGTGTGCGTACTG CTGCAATAT (SEQ ID NO: 13) |
| SARS-CoV-1 | Envelope protei n | E | GCTGCAATATTGTTAACGTGAGTTTAGTAAAACCA ACGGTTTACGTCTACTCGCGTGTTAAAAATCTGAA CTCTTCTGAAGGAGTTCC |
| | | | (SEQ ID NO: 14) |
| MERS-CoV | Nucleocapsid | N2 | GGCACTGAGGACCCACGTTGGCCCCAAATTGCTG AGCTTGCTCCTACAGCCAGTGCTTTTATGGGTAT GTCGCAA (SEQ ID NO: 15) |

**Table 3. Cas9 proteins used.**

| **Cas protein** | **Sequence 5' - 3'** |
|---|---|
| Wild type Cas9 nuclease |  (SEQ ID NO: 16) |
| Cas9 H840A nickase | MDKKYSIGLDIGTNSVGWAVITDEYKVPSKKFKVLGNTDRHSIKKNLIGAL LFDSGETAEATRLKRTARRRYTRRKNRICYLQEIFSNEMAKVDDSFFHRL EESFLVEEDKKHERHPIFGNIVDEVAYHEKYPTIYHLRKKLVDSTDKADLR LIYLALAHMIKFRGHFLIEGDLNPDNSDVDKLFIQLVQTYNQLFEENPINAS GVDAKAILSARLSKSRRLENLIAQLPGEKKNGLFGNLIALSLGLTPNFKSN FDLAEDAKLQLSKDTYDDDLDNLLAQIGDQYADLFLAAKNLSDAILLSDILR VNTEITKAPLSASMIKRYDEHHQDLTLLKALVRQQLPEKYKEIFFDQSKNG |
| | YAGYIDGGASQEEFYKFIKPILEKMDGTEELLVKLNREDLLRKQRTFDNG SIPHQIHLGELHAILRRQEDFYPFLKDNREKIEKILTFRIPYYVGPLARGNS RFAWMTRKSEETITPWNFEEVVDKGASAQSFIERMTNFDKNLPNEKVLP KHSLLYEYFTVYNELTKVKYVTEGMRKPAFLSGEQKKAIVDLLFKTNRKV TVKQLKEDYFKKIECFDSVEISGVEDRFNASLGTYHDLLKIIKDKDFLDNEE NEDILEDIVLTLTLFEDREMIEERLKTYAHLFDDKVMKQLKRRRYTGWGRL SRKLINGIRDKQSGKTILDFLKSDGFANRNFMQLIHDDSLTFKEDIQKAQV SGQGDSLHEHIANLAGSPAIKKGILQTVKVVDELVKVMGRHKPENIVIEMA RENQTTQKGQKNSRERMKRIEEGIKELGSQILKEHPVENTQLQNEKLYLY YLQNGRDMYVDQELDINRLSDYDVDAIVPQSFLKDDSIDNKVLTRSDKNR GKSDNVPSEEVVKKMKNYWRQLLNAKLITQRKFDNLTKAERGGLSELDK AGFIKRQLVETRQITKHVAQILDSRMNTKYDENDKLIREVKVITLKSKLVSD FRKDFQFYKVREINNYHHAHDAYLNAVVGTALIKKYPKLESEFVYGDYKV YDVRKMIAKSEQEIGKATAKYFFYSNIMNFFKTEITLANGEIRKRPLIETNG ETGEIVWDKGRDFATVRKVLSMPQVNIVKKTEVQTGGFSKESILPKRNSD KLIARKKDWDPKKYGGFDSPTVAYSVLVVAKVEKGKSKKLKSVKELLGITI MERSSFEKNPIDFLEAKGYKEVKKDLIIKLPKYSLFELENGRKRMLASAGE LQKGNELALPSKYVNFLYLASHYEKLKGSPEDNEQKQLFVEQHKHYLDEI IEQISEFSKRVILADANLDKVLSAYNKHRDKPIREQAENIIHLFTLTNLGAPA AFKYFDTTIDRKRYTSTKEVLDATLIHQSITGLYETRIDLSQLGGD (SEQ ID NO: 17) |
| Catalytically dead Cas9 | MDKKYSIGLAIGTNSVGWAVITDEYKVPSKKFKVLGNTDRHSIKKNLIGAL LFDSGETAEATRLKRTARRRYTRRKNRICYLQEIFSNEMAKVDDSFFHRL EESFLVEEDKKHERHPIFGNIVDEVAYHEKYPTIYHLRKKLVDSTDKADLR LIYLALAHMIKFRGHFLIEGDLNPDNSDVDKLFIQLVQTYNQLFEENPINAS GVDAKAILSARLSKSRRLENLIAQLPGEKKNGLFGNLIALSLGLTPNFKSN FDLAEDAKLQLSKDTYDDDLDNLLAQIGDQYADLFLAAKNLSDAILLSDILR VNTEITKAPLSASMIKRYDEHHQDLTLLKALVRQQLPEKYKEIFFDQSKNG YAGYIDGGASQEEFYKFIKPILEKMDGTEELLVKLNREDLLRKQRTFDNG SIPHQIHLGELHAILRRQEDFYPFLKDNREKIEKILTFRIPYYVGPLARGNS RFAWMTRKSEETITPWNFEEVVDKGASAQSFIERMTNFDKNLPNEKVLP KHSLLYEYFTVYNELTKVKYVTEGMRKPAFLSGEQKKAIVDLLFKTNRKV TVKQLKEDYFKKIECFDSVEISGVEDRFNASLGTYHDLLKIIKDKDFLDNEE NEDILEDIVLTLTLFEDREMIEERLKTYAHLFDDKVMKQLKRRRYTGWGRL SRKLINGIRDKQSGKTILDFLKSDGFANRNFMQLIHDDSLTFKEDIQKAQV SGQGDSLHEHIANLAGSPAIKKGILQTVKVVDELVKVMGRHKPENIVIEMA RENQTTQKGQKNSRERMKRIEEGIKELGSQILKEHPVENTQLQNEKLYLY YLQNGRDMYVDQELDINRLSDYDVDAIVPQSFLKDDSIDNKVLTRSDKNR GKSDNVPSEEVVKKMKNYWRQLLNAKLITQRKFDNLTKAERGGLSELDK AGFIKRQLVETRQITKHVAQILDSRMNTKYDENDKLIREVKVITLKSKLVSD FRKDFQFYKVREINNYHHAHDAYLNAVVGTALIKKYPKLESEFVYGDYKV YDVRKMIAKSEQEIGKATAKYFFYSNIMNFFKTEITLANGEIRKRPLIETNG ETGEIVWDKGRDFATVRKVLSMPQVNIVKKTEVQTGGFSKESILPKRNSD KLIARKKDWDPKKYGGFDSPTVAYSVLVVAKVEKGKSKKLKSVKELLGITI MERSSFEKNPIDFLEAKGYKEVKKDLIIKLPKYSLFELENGRKRMLASAGE LQKGNELALPSKYVNFLYLASHYEKLKGSPEDNEQKQLFVEQHKHYLDEI |
| | IEQISEFSKRVILADANLDKVLSAYNKHRDKPIREQAENIIHLFTLTNLGAPA AFKYFDTTIDRKRYTSTKEVLDATLIHQSITGLYETRIDLSQLGGD (SEQ ID NO: 18) |

**Table 4. sgRNAs used.**

| **Virus** | **Gene** | **Region** | **Sequence 5' - 3'** |
|---|---|---|---|
| SARS-CoV-2 | Nucleocapsid | N1 | GGAAAAUUAGCGAAAUGCACCCCGCAUUACGUU GUUUUAGAGCUAGAAAUAGCAAGUUAAAAUAAG GCUAGUCCGUUAUCAACUUGAAAAAGUGGCACC GAGUCGGUGCUUUU (SEQ ID NO: 19) |
| SARS-CoV-2 | Nucleocapsid | N2 | GGCAUUUCGAAGAACGCUGAAGCGCGUUUUAGA GCUAGAAAUAGCAAGUUAAAAUAAGGCUAGUCC GUUAUCAACUUGAAAAAGUGGCACCGAGUCGGU GCUUUU (SEQ ID NO: 20) |
| SARS-CoV-2 | Envelope protein | E | GGAGUAGUACGCACACAAUCGAAGCGCAGUAGU UUUAGAGCUAGAAAUAGCAAGUUAAAAUAAGGC UAGUCCGUUAUCAACUUGAAAAAGUGGCACCGA GUCGGUGCUUUU (SEQ ID NO: 21) |
| SARS-CoV-1 | Envelope protein | E | GGAUAUUGUUAAUGUGAGUUUAGUAAAACCAAG UUUUAGAGCUAGAAAUAGCAAGUUAAAAUAAGG CUAGUCCGUUAUCAACUUGAAAAAGUGGCACCG AGUCGGUGCUUUU (SEQ ID NO: 22) |
| MERS-CoV | Nucleocapsid | N2 | GGCAUAUCCAUAAAAGCACUGGCUGUGUUUUAG AGCUAGAAAUAGCAAGUUAAAAUAAGGCUAGUC CGUUAUCAACUUGAAAAAGUGGCACCGAGUCGG UGCUUUU (SEQ ID NO: 23) |

**Table 5. Molecular bacons used.**

| **Virus** | **Gene** | **(5'-) Quencher** | **Fluorophore (-3')** | **Sequence 5' - 3'** |
|---|---|---|---|---|
| SARS-CoV-2 | Nucleocapsid N1 | Iowa Black FQ | FAM | CGCTGATTTTGGGGTCGTAG TTAATCAGCGA (SEQ ID NO: 24) |
| SARS-CoV-2 | Nucleocapsid N2 | Iowa Black FQ | TAMRA | CTTCGGAATGTCGCGCGCTT TCTTCCGAAGT (SEQ ID NO: 25) |
| SARS-CoV-2 | Envelope protein | Iowa Black RQ | CY5 | GCGTACTGCTGCAATATTCG TCCAGTACGC (SEQ ID NO: 26) |
| SARS-CoV-1 | Envelope protein | Iowa Black FQ | TAMRA | CTCACGTTAACAATATTGCA GCGTCATCATATTGTTAATGT GAGT (SEQ ID NO: 27) |
| MERS-CoV | Nucleocapsid | Iowa Black RQ | CY5 | GGCTTTTATGGGTATGTCGC AATATTATTACCTATAAAGC C (SEQ ID NO: 28) |

**Table 6. Viral nucleotide sequences.**

| **Virus** | **Gene** | **Region** | **Sequence 5' - 3'** |
|---|---|---|---|
| SARS-CoV-2 | Nucleocapsid | N1 | GACCCCAAAAUCAGCGAAAUGCACCCCGCAUUA CGUUUGGUGGACCCUCAGAUUCAACUGGCAGUA ACCAGA (SEQ ID NO: 29) |
| SARS-CoV-2 | Nucleocapsid | N2 | UUACAAACAUUGGCCGCAAAUUGCACAAUUUGC CCCCAGCGCUUCAGCGUUCUUCGGAAUGUCGC GC (SEQ ID NO: 30) |
| SARS-CoV-2 | Envelope protei n | E | ACAGGUACGUUAAUAGUUAAUAGCGUACUUCUU UUUCUUGCUUUCGUGGUAUUCUUGCUAGUUACA CUAGCCAUCCUUACUGCGCUUCGAUUGUGUGCG UACUGCUGCAAUAU (SEQ ID NO: 31) |
| SARS-CoV-1 | Envelope protei n | E | GCUGCAAUAUUGUUAACGUGAGUUUAGUAAAAC CAACGGUUUACGUCUACUCGCGUGUUAAAAAUC UGAACUCUUCUGAAGGAGUUCC (SEQ ID NO: 32) |
| MERS-CoV | Nucleocapsid | N2 | GGCACUGAGGACCCACGUUGGCCCCAAAUUGCU GAGCUUGCUCCUACAGCCAGUGCUUUUAUGGGU AUGUCGCAA (SEQ ID NO: 33) |

## Claims

1. Method for *in vitro* virus detection and/or *in vitro* specific viral sequence detection, comprising the following steps:
(a) amplifying, at least, one target viral nucleotide sequence from a sample, obtaining at least one amplicon with a protospacer adjacent motif (PAM), preferably wherein the sample is an isolated biological sample,
(b) contacting the amplicon obtained in the step (a) to a Cas9 protein and, at least, one single guide RNA (sgRNA), wherein the sgRNA comprises a nucleotide sequence that hybridizes with a target region of the amplicon obtained in (a), obtaining at least one ribonucleoprotein-DNA complex,
(c) adding, at least, one molecular beacon to the ribonucleoprotein-DNA complex obtained in (b), wherein the molecular beacon comprises a nucleotide sequence that hybridizes with a strand-displaced region of the amplicon, a fluorophore linked to the 3' or 5' end of its nucleotide sequence, and a quencher linked to the remaining end of its nucleotide sequence, arranged in a conformation, such that:
(i) when the molecular beacon does not hybridize to the strand-displaced region of the amplicon, the molecular beacon is arranged in a hairpin structure, with the quencher and fluorophore located spatially close without emitting fluorescence or emitting a basal fluorescence;
(ii) when the molecular beacon hybridizes to the strand-displaced region of the amplicon, the molecular beacon is arranged linearly, with the quencher and fluorophore being spatially separated, emitting fluorescence, and
(d) measuring, at least, one fluorescent signal and comparing to control values, wherein an increase of the fluorescent signal compared to control values indicates that the target viral nucleotide sequence is present in the sample, hence allowing virus detection and/or specific viral sequence detection.

2. The method according to claim 1, wherein the Cas9 protein is from *Streptococcus pyogenes.*

3. The method according to claim 2, wherein the *Streptococcus pyogenes* Cas9 is selected from the list consisting of: wild type Cas9 nuclease, Cas9 H840A nickase and catalytically dead Cas9.

4. The method according to any one of claims 1 to 3, wherein the target viral nucleotide sequence is amplified in step (a) by PCR, recombinase polymerase amplification (RPA) or loop-mediated isothermal amplification (LAMP).

5. The method according to any one of claims 1 to 4, wherein the isolated biological sample is from a subject infected with, at least, one respiratory virus, preferably wherein the respiratory virus is selected from the list consisting of SARS-CoV-2, SARS-CoV-1, MERS-CoV, and any combination thereof.

6. The method according to any one of claims 1 to 5, wherein virus detection comprises the detection of, at least, one virus.

7. The method according to claim 6, wherein virus detection comprises the simultaneous detection of, at least, 2 viruses.

8. The method according to claim 6 or 7, wherein the virus is a respiratory virus, preferably a coronavirus.

9. The method according to claim 8, wherein the coronavirus is selected from the list consisting of SARS-CoV-2, SARS-CoV-1, MERS-CoV, and any combination thereof.

10. The method according to any one of claims 1 to 5, wherein the specific viral sequence detection comprises the detection of, at least, one specific viral sequence.

11. The method according to claim 10, wherein the specific viral sequence detection comprises the simultaneous detection of, at least, 2 specific viral sequences.

12. The method according to claim 10 or 11, wherein the viral sequence is from, at least, one coronavirus, preferably wherein the coronavirus is selected from the list consisting of SARS-CoV-2, SARS-CoV-1, MERS-CoV, and any combination thereof.

13. The method according to claim 12, wherein the specific viral sequence is selected from the list of sequences consisting of SEQ ID NO: 31, SEQ ID NO: 29, SEQ ID NO: 30, SEQ ID NO: 32, SEQ ID NO: 33, and any combination thereof.

14. A kit comprising:
- a Cas9 protein, preferably wherein the Cas9 protein is from *Streptococcus pyogenes,*
- at least, one single sgRNA, wherein the sgRNA comprises a nucleotide sequence that hybridizes with a target region of a viral amplicon, and
- at least, one molecular beacon, wherein the molecular beacon comprises a nucleotide sequence that hybridizes with a strand-displaced region of the viral amplicon, a fluorophore linked to the 3' or 5' end of its nucleotide sequence, and a quencher linked to the remaining end of its nucleotide sequence, arranged in a conformation, such that:
(i) when the molecular beacon does not hybridize with the strand-displaced region of the viral amplicon, the molecular beacon is arranged in a hairpin structure, with the quencher and fluorophore located spatially close without emitting fluorescence or emitting a basal fluorescence;
(ii) when the molecular beacon hybridizes with the strand-displaced region of the viral amplicon, the molecular beacon is arranged linearly, with the quencher and fluorophore being spatially separated, emitting fluorescence.

15. The kit according to claim 14, further comprising the means necessary for carrying out the method of the invention according to claims 1 to 13.
